# EUROPEAN PATENT SPECIFICATION

(11) **EP 1 713 934 B1**
(45) Date of publication and mention of the grant of the patent: **02.03.2011**
(21) Application number: 05708322.2
(22) Date of filing: 11.02.2005
(51) Int. Cl.: C12Q 1/68, C07H 21/04

(54) **TB RESISTANCE ASSAY**
TB-RESISTENZ-TEST
DOSAGE DE RESISTANCE A TB

(30) Priority: 11.02.2004 GB 0403039
(43) Date of publication of application: 25.10.2006
(73) Proprietor: Health Protection Agency, 61 Colindale Avenue London NW9 5DF (GB)
(72) Inventor: BROWN, Timothy, HPA Mycobacterium Reference Unit, 2 Newark Street, London E1 2 AT (GB)
(74) Representative: Mathys & Squire LLP
(86) International application number: PCT/GB2005/000499
(87) International publication number: WO 2005/078131

(56) References cited:
- WO-A-03/031654
- US-A- 5 658 733
- US-B1- 6 228 575
- NIKOLAYEVSKY V ET AL: "Detection of mutations associated with isoniazid and rifampin resistance in Mycobacterium tuberculosis isolates from Samara Region, Russian Federation." JOURNAL OF CLINICAL MICROBIOLOGY. OCT 2004, vol. 42, no. 10, October 2004 (2004-10), pages 4498-4502, XP002330872 ISSN: 0095-1137
- MOKROUSOV IGOR ET AL: "Multicenter evaluation of reverse line blot assay for detection of drug resistance in Mycobacterium tuberculosis clinical isolates." JOURNAL OF MICROBIOLOGICAL METHODS. JUN 2004, vol. 57, no. 3, June 2004 (2004-06), pages 323-335, XP002330873 ISSN: 0167-7012
- TORRES MARIA J ET AL: "Improved real-time PCR for rapid detection of rifampin and isoniazid resistance in Mycobacterium tuberculosis clinical isolates." DIAGNOSTIC MICROBIOLOGY AND INFECTIOUS DISEASE, vol. 45, no. 3, March 2003 (2003-03), pages 207-212, XP002330874 ISSN: 0732-8893
- SKOTNIKOVA O I ET AL: "Typing of Mycobacterium tuberculosis strains resistant to rifampicin and isoniazid by molecular biological methods." BULLETIN OF EXPERIMENTAL BIOLOGY AND MEDICINE. SEP 2003, vol. 136, no. 3, September 2003 (2003-09), pages 273-275, XP009048647 ISSN: 0007-4888
- TELENTI A ET AL: "Genotypic assessment of isoniazid and rifampin resistance in Mycobacterium tuberculosis: a blind study at reference laboratory level." JOURNAL OF CLINICAL MICROBIOLOGY. MAR 1997, vol. 35, no. 3, March 1997 (1997-03), pages 719-723, XP002330875 ISSN: 0095-1137

### SEQUENCE LISTING

<110> Health Protection Agency
<120> TB Resistance Assay
<130> P26141WO-MRM/PJG
<150> GB 0403039.1
   <151> 2004-02-11
<160> 78
<170> PatentIn version 3.2
<210> 1
   <211> 21
   <212> DNA
   <213> artificial sequence
<220>
   <223> probe
<400> 1
   ctcgatgccg ctggtgatcg c 21
<210> 2
   <211> 21
   <212> DNA
   <213> artificial sequence
<220>
   <223> probe
<400> 2
   gcgatcacca ccggcatcga 21
<210> 3
   <211> 21
   <212> DNA
   <213> artificial sequence
<220>
   <223> probe
<400> 3
   ggcgagacga taggttgtcg g 21
<210> 4
   <211> 21
   <212> DNA
   <213> artificial sequence
<220>
   <223> probe
<400> 4
   ggcgagatga taggttgtcg g 21
<210> 5
   <211> 23
   <212> DNA
   <213> artificial sequence
<220>
   <223> probe
<400> 5
   gccagctgag ccaattcatg gac 23
<210> 6
   <211> 24
   <212> DNA
   <213> artificial sequence
<220>
   <223> probe
<400> 6
   gccaattcat ggaccagaac aacc 24
<210> 7
   <211> 22
   <212> DNA
   <213> artificial sequence
<220>
   <223> probe
<400> 7
   tggaccagaa caacccgctg tc 22
<210> 8
   <211> 22
   <212> DNA
   <213> artificial sequence
<220>
   <223> probe
<400> 8
   acaacccgct gtcggggttg ac 22
<210> 9
   <211> 21
   <212> DNA
   <213> artificial sequence
<220>
   <223> probe
<400> 9
   ggttgaccca caagcgccga c µ21
<210> 10
   <211> 23
   <212> DNA
   <213> artificial sequence
<220>
   <223> probe
<400> 10
   cgactgtcgg cactggggcc cgg 23
<210> 11
   <211> 23
   <212> DNA
   <213> artificial sequence
<220>
   <223> probe
<400> 11
   cagccagcca gctgagccaa ttc 23
<210> 12
   <211> 23
   <212> DNA
   <213> artificial sequence
<220>
   <223> probe
<400> 12
   ccagccagct gagccaattc atg 23
<210> 13
   <211> 23
   <212> DNA
   <213> artificial sequence
<220>
   <223> probe
<400> 13
   agctgagcca attcatggac cag 23
<210> 14
   <211> 24
   <212> DNA
   <213> artificial sequence
<220>
   <223> probe
<400> 14
   tgagccaatt catggaccag aaca 24
<210> 15
   <211> 24
   <212> DNA
   <213> artificial sequence
<220>
   <223> probe
<400> 15
   aattcatgga ccagaacaac ccgc 24
<210> 16
   <211> 23
   <212> DNA
   <213> artificial sequence
<220>
   <223> probe
<400> 16
   tcatggacca gaacaacccg ctg 23
<210> 17
   <211> 22
   <212> DNA
   <213> artificial sequence
<220>
   <223> probe
<400> 17
   accagaacaa cccgctgtcg gg 22
<210> 18
   <211> 22
   <212> DNA
   <213> artificial sequence
<220>
   <223> probe
<400> 18
   agaacaaccc gctgtcgggg tt 22
<210> 19
   <211> 21
   <212> DNA
   <213> artificial sequence
<220>
   <223> probe
<400> 19
   acccgctgtc ggggttgacc c 21
<210> 20
   <211> 22
   <212> DNA
   <213> artificial sequence
<220>
   <223> probe
<400> 20
   cgctgtcggg gttgacccac aa 22
<210> 21
   <211> 22
   <212> DNA
   <213> artificial sequence
<220>
   <223> probe
<400> 21
   tgtcggggtt gacccacaag cg 22
<210> 22
   <211> 21
   <212> DNA
   <213> artificial sequence
<220>
   <223> probe
<400> 22
   cggggttgac ccacaagcgc c 21
<210> 23
   <211> 22
   <212> DNA
   <213> artificial sequence
<220>
   <223> probe
<400> 23
   tgacccacaa gcgccgactg tc 22
<210> 24
   <211> 21
   <212> DNA
   <213> artificial sequecne
<220>
   <223> probe
<400> 24
   cccacaagcg ccgactgtcg g 21
<210> 25
   <211> 21
   <212> DNA
   <213> artificial sequence
<220>
   <223> probe
<400> 25
   acaagcgccg actgtcggcg c 21
<210> 26
   <211> 21
   <212> DNA
   <213> artificial sequence
<220>
   <223> probe
<400> 26
   agcgccgact gtcggcgctg g 21
<210> 27
   <211> 21
   <212> DNA
   <213> artificial sequence
<220>
   <223> probe
<400> 27
   gccgactgtc ggcgctgggg c 21
<210> 28
   <211> 21
   <212> DNA
   <213> artificial sequence
<220>
   <223> probe
<400> 28
   gactgtcggc gctggggccc g 21
<210> 29
   <211> 20
   <212> DNA
   <213> artificial sequence
<220>
   <223> probe
<400> 29
   tgtcggcgct ggggcccggc 20
<210> 30
   <211> 20
   <212> DNA
   <213> artificial sequence
<220>
   <223> prob
<400> 30
   cggcgctggg gcccggcggt 20
<210> 31
   <211> 20
   <212> DNA
   <213> artificial sequence
<220>
   <223> probe
<400> 31
   cgctggggcc cggcggtctg 20
<210> 32
   <211> 21
   <212> DNA
   <213> artificial sequence
<220>
   <223> probe
<400> 32
   tggggcccgg cggtctgtca c 21
<210> 33
   <211> 21
   <212> DNA
   <213> artificial sequence
<220>
   <223> forward primer
<400> 33
   cgctggagca gatgggcttg g 21
<210> 34
   <211> 21
   <212> DNA
   <213> artificial sequence
<220>
   <223> reverse primer
<400> 34
   gtcagctccc actcgtagcc g 21
<210> 35
   <211> 21
   <212> DNA
   <213> artificial sequence
<220>
   <223> forward primer
<400> 35
   gcagccacgt tacgctcgtg g 21
<210> 36
   <211> 21
   <212> DNA
   <213> artificial sequence
<220>
   <223> reverse primer
<400> 36
   cgatcccccg gtttcctccg g 21
<210> 37
   <211> 20
   <212> DNA
   <213> artificial sequence
<220>
   <223> forward primer
<400> 37
   ggtcggcatg tcgcggatgg 20
<210> 38
   <211> 21
   <212> DNA
   <213> artificial sequence
<220>
   <223> reverse primer
<400> 38
   gtagtgcgac gggtgcacgt c 21
<210> 39
   <211> 10
   <212> DNA
   <213> mycobacterium tuberculosis
<400> 39
   accagcggca 10
<210> 40
   <211> 10
   <212> DNA
   <213> mycobacterium tuberculosis
<400> 40
   gccggtggtg 10
<210> 41
   <211> 10
   <212> DNA
   <213> mycobacterium tuberculosis
<400> 41
   tatcgtctcg 10
<210> 42
   <211> 10
   <212> DNA
   <213> mycobacterium tuberculosis
<400> 42
   tatcatctcg 10
<210> 43
   <211> 10
   <212> DNA
   <213> mycobacterium tuberculosis
<400> 43
   gaattggctc 10
<210> 44
   <211> 10
   <212> DNA
   <213> mycobacterium tuberculosis
<400> 44
   ctggtccatg 10
<210> 45
   <211> 10
   <212> DNA
   <213> mycobacterium tuberculosis
<400> 45
   ggttgttctg 10
<210> 46
   <211> 10
   <212> DNA
   <213> mycobacterium tuberculosis
<400> 46
   cccgacagcg 10
<210> 47
   <211> 10
   <212> DNA
   <213> mycobacterium tuberculosis
<400> 47
   gcttgtgggt 10
<210> 48
   <211> 10
   <212> DNA
   <213> mycobacterium tuberculosis
<400> 48
   ccagtgccga 10
<210> 49
   <211> 15
   <212> DNA
   <213> mycobacterium tuberculosis
<400> 49
   atcaccagcg gcatc 15
<210> 50
   <211> 15
   <212> DNA
   <213> mycobacterium tuberculosis
<400> 50
   gatgccggtg gtgta 15
<210> 51
   <211> 15
   <212> DNA
   <213> mycobacterium tuberculosis
<400> 51
   acctatcgtc tcgcc 15
<210> 52
   <211> 15
   <212> DNA
   <213> mycobacterium tuberculosis
<400> 52
   acctatcatc tcgcc 15
<210> 53
   <211> 15
   <212> DNA
   <213> mcyobacterium tuberculosis
<400> 53
   atgaattggc tcagc 15
<210> 54
   <211> 15
   <212> DNA
   <213> mycobacterium tuberculosis
<400> 54
   gttctggtcc atgaa 15
<210> 55
   <211> 15
   <212> DNA
   <213> mycobacterium tuberculosis
<400> 55
   gcgggttgtt ctggt 15
<210> 56
   <211> 14
   <212> DNA
   <213> mycobacterium tuberculosis
<400> 56
   aaccccgaca gcgg 14
<210> 57
   <211> 15
   <212> DNA
   <213> mycobacterium tuberculosis
<400> 57
   ggcgcttgtg ggtca 15
<210> 58
   <211> 15
   <212> DNA
   <213> mycobacterium tuberculosis
<400> 58
   gccccagtgc cgaca 1
<210> 59
   <211> 10
   <212> DNA
   <213> artificial sequence
<220>
   <223> prob
<400> 59
   tgccgctggt 10
<210> 60
   <211> 10
   <212> DNA
   <213> artificial sequence
<220>
   <223> probe
<400> 60
   caccaccggc 10
<210> 61
   <211> 10
   <212> DNA
   <213> artificial sequence
<220>
   <223> probe
<400> 61
   cgagacgata 10
<210> 62
   <211> 10
   <212> DNA
   <213> artificial sequence
<220>
   <223> probe
<400> 62
   cgagatgata 10
<210> 63
   <211> 10
   <212> DNA
   <213> artificial sequence
<220>
   <223> probe
<400> 63
   gagccaattc 10
<210> 64
   <211> 10
   <212> DNA
   <213> artificial sequence
<220>
   <223> probe
<400> 64
   catggaccag 10
<210> 65
   <211> 10
   <212> DNA
   <213> artificial sequence
<220>
   <223> probe
<400> 65
   cagaacaacc 10
<210> 66
   <211> 10
   <212> DNA
   <213> artificial sequence
<220>
   <223> probe
<400> 66
   cgctgtcggg 10
<210> 67
   <211> 10
   <212> DNA
   <213> artificial sequence
<220>
   <223> probe
<400> 67
   acccacaagc 10
<210> 68
   <211> 10
   <212> DNA
   <213> artificial sequence
<220>
   <223> probe
<400> 68
   tcggcactgg 10
<210> 69
   <211> 15
   <212> DNA
   <213> artificial sequence
<220>
   <223> probe
<400> 69
   gatgccgctg gtgat 15
<210> 70
   <211> 15
   <212> DNA
   <213> artificial sequence
<220>
   <223> probe
<400> 70
   atcaccaccg gcatc 15
<210> 71
   <211> 15
   <212> DNA
   <213> artificial sequence
<220>
   <223> probe
<400> 71
   ggcgagacga taggt 15
<210> 72
   <211> 15
   <212> DNA
   <213> artificial sequence
<220>
   <223> probe
<400> 72
   ggcgagatga taggt 15
<210> 73
   <211> 17
   <212> DNA
   <213> artificial sequence
<220>
   <223> probe
<400> 73
   agctgagcca attcatg 17
<210> 74
   <211> 18
   <212> DNA
   <213> artificial sequence
<220>
   <223> probe
<400> 74
   aattcatgga ccagaaca 18
<210> 75
   <211> 15
   <212> DNA
   <213> artificial sequence
<220>
   <223> probe
<400> 75
   accagaacaa cccgc 15
<210> 76
   <211> 16
   <212> DNA
   <213> artificial sequence
<220>
   <223> probe
<400> 76
   acccgctgtc ggggtt 16
<210> 77
   <211> 15
   <212> DNA
   <213> artificial sequence
<220>
   <223> probe
<400> 77
   tgacccacaa gcgcc 15
<210> 78
   <211> 17
   <212> DNA
   <213> artificial sequence
<220>
   <223> probe
<400> 78
   ctgtcggcac tggggcc 17

## Description

The present invention relates to a diagnostic assay for multi-drug resistant *Mycobacterium sp.,* in particular *Mycobacterium tuberculosis,* and to reagents and kits therefor.

*Mycobacterium tuberculosis* and closely related species make up a small group of mycobacteria known as the *Mycobacterium tuberculosis* complex (MTC). This group comprises five species - *M. tuberculosis, M. microti, M. bovis, M. caneti,* and *M. africanum -* which are the causative agent in the majority of cases of *Mycobacterium tuberculosis* infection (TB) throughout the world.

*M. tuberculosis* is responsible for more than three million deaths a year worldwide. The WHO estimates that up to a third of the world's population is infected with *Mycobacterium tuberculosis* and globally someone dies of TB every 15 seconds. Other mycobacteria are also pathogenic in man and animals, for example *M. avium* subsp. *paratuberculosis* which causes Johne's disease in ruminants, *M. bovis* which causes tuberculosis in cattle, *M. avium* and *M. intracellulare* which cause tuberculosis in immunocompromised patients (eg. AIDS patients, and bone marrow transplant patients), and *M. leprae* which causes leprosy in humans. Another important mycobacterial species is *M. vaccae.*

Epidemiological data shows high incidence rates of *Mycobacterium sp.* infection, including MTC infection such as *Mycobacterium tuberculosis* infection, across all former Soviet Union countries. In Russia, a TB incidence of 90.7/100,000 was recorded in 2000. Similarly high rates of drug resistance, particularly multiple drug resistance caused by multiple drug resistant *Mycobacterium tuberculosis* (MDRTB), have been reported, contributing to low cure rates and compromising the effectiveness of TB. programmes. Rates of MDRTB as high as 14% of newly diagnosed cases have been reported in Estonia. In 2000, rates of primary and secondary resistance to at least one drug in North Russia were reported to be 33% and 85%, respectively, in the Archangelsk oblast. High drug resistance rates were reported in Samara and St Petersburg. Conversely, although large institutional outbreaks of drug resistant and MDRTB have been reported in the USA, the UK and other European countries the overall MDRTB incidence and prevalence are low.

A number of factors have contributed to the problem of microbial resistance. One is the accumulation of mutations over time and the subsequent horizontal and vertical transfer of the mutated genes to other organisms. Thus, for a given pathogen, entire classes of antibiotics have been rendered inactive. A further factor has been the absence of a new class of antibiotics in recent years. The emergence of multiple drug-resistant pathogenic bacteria such as *Mycobacterium sp.,* in particular those species of the MTC, such as *Mycobacterium tuberculosis,* represents a serious threat to public health and new forms of therapy are urgently required.

Multi-drug resistance in *Mycobacterium sp.* such as *Mycobacterium tuberculosis (M. tuberculosis)* is assessed in relation to two drugs - rifampin and isoniazid. Multidrug-resistant (MDR) strains which are defined as being resistant to INH and RIF are emerging and their involvement in several outbreaks has been reported. Currently available methods for detection of drug resistance include sputum microscopy, and growth based methods that measure growth of bacteria on solid or liquid media (usually Lowenstein-Jensen media) in the presence of drugs - "Drug Susceptibility Testing" (DST). A disadvantage of growth-based techniques is that they are limited by the growth rate of *Mycobacterium sp.* such as *M. tuberculosis,* which has a doubling time of 16 hours (compare *E. coli*, which has a doubling time of 20 minutes). Hence, it typically takes at least 10-14 days (typically 21-30 days after the primary culture has been isolated) to identify drug resistant *Mycobacterium sp.* by this method. The use of non-standardised methods also compromises the accuracy of DST.

The viability of *Mycobacterium sp.* such as *M. tuberculosis* after exposure to drugs (and hence, drug resistance) can also be monitored by studying the ability of mycobacteriophage to successfully infect the organism. Two methods have been described. In one method, *Mycobacterium sp.* such as *M. tuberculosis* are treated with a drug, and then phage are used to infect the drug-treated *M. tuberculosis.*

Extracellular phage are destroyed before the *M. tuberculosis* are lysed, and the phage are enumerated on a lawn of rapidly growing mycobacteria. The number of plaques is proportional to the number of viable *M. tuberculosis.* In the second method, a luminescent phage is used to infect drug-exposed *Mycobacterium sp.* such as *M. tuberculosis,* the luminescence being proportional to the number of viable *M. tuberculosis.* A disadvantage of each of these methods is that they both take approximately 2 days for identification of drug resistant *Mycobacterium sp.*

At least 11 genes have been reported to be involved in the development of resistance to the main anti-TB drugs. The detection of rifampin resistance (RIF resistance) or isoniazid resistance (INH resistance), is of importance clinically and for public health TB control.

Resistance to rifampin and isoniazid is conferred by mutations in three genes. RIF resistance is generally associated with single nucleotide substitutions. Mutations in the 81 bp core region of the *rpoB* gene (encoding the ß-subunit of RNA polymerase) are known to be responsible for over 90% of RIF resistance - approximately 60-70% of mutations are found within two codons, 531 and 526.

Mutations in two different genes are known to be responsible for resistance to isoniazid. In more than 75% of cases, INH resistance occurs due to substitutions in the *katG* gene (encoding catalase-peroxidase), particularly at codon 315 (AGC-ACC). More rarely, INH resistance is due to mutations in the *inhA* and *ahpC* genes.

The prevalence of mutations at codon 315 of the *katG* gene varies depending on the geographical region studied with percentages from 35% in Beirut to over 90% in Latvia and Russia, while the prevalence of the *inh*A mutation varies geographically from 3.3% to over 32%.

Specific single mutations associated with INH or RIF resistance may be detected in less than 1 day using PCR amplification of *Mycobacterium sp.* nucleic acid, such as *M. tuberculosis* nucleic acid, followed by DNA sequence analysis.

Various methods for sequence analysis of specific, single *Mycobacterium sp.* mutations have been employed in the art, such as agarose gel electrophoresis. This method requires mutation specific amplification - the size of the resultant PCR product in a gel indicating the presence of any given mutation. Another method for sequence analysis is SSCP (single strand conformation polymorphism analysis), in which a region of DNA containing a given mutation is amplified by PCR, then this PCR product is denatured, and the resultant single stranded DNA is passed down an acrylamide gel - a typical migration pattern being seen with each mutation. A further technique for sequence analysis is melting curve analysis. Melt curves can be generated using Real-time PCR equipment such as a LightCycler (Roche), and each mutation will have a typical curve. Mutations in PCR products can also be identified using fluorescent probes. Nucleic acid sequences can also be determined using commercially available sequencing equipment.

A disadvantage of all the above sequencing techniques is that they cannot be multiplexed to a degree that allows all DNA analysis to take place in a single test. Although it may, theoretically, be possible to identify a number of mutant sites using a LightCycler PCR assay, in practice there are problems caused by cross-talk when a number of different dyes are used. Hence these known methods do not enable all of the mutant target sites to be identified simultaneously.

An alternative method for sequence analysis is reverse hybridisation. A labelled PCR product is generated that includes the mutation of interest, and this is used to interrogate a series of probes immobilised on a solid support. A system for detecting mutations in *rpoB* associated with RIF resistance is commercially available (INNO-LiPA Rif.TB, Innogenetics, Gent, Belgium). The application of this kit for screening purposes is, however, limited in regions with high TB incidence and high rates of MDRTB, due to a relatively high cost and impossibility to analyse INH resistance.

Non-commercial dot-blot strategies, based on amplification of gene fragments known to confer INH resistance, followed by hybridisation with mutant and wild-type oligonucleotide probes, have been found to be a more cost-effective methodology, predicting RIF resistance in 90% of cases or INH resistance in 75% of cases.

There is, therefore, a need to provide an alternative and/or improved system for detecting multi-drug resistant *Mycobacterium sp.,* in particular those members of the MTC, such as multi-drug resistant *M. tuberculosis* (MDRTB). This need is fulfilled by the present invention, which solves one or more of the above defined technical problems.

US 5,658,733 describes a method for detection of isoniazid resistant strains of *M. tuberculosis.*

Torres, M. et al., Diagnostic Microbiology and Infectious Disease (2003) Vol. 45, pages 207-212, describes a real-time PCR for rapid detection of rifampin and isoniazid resistance in *M. tuberculosis* clinical isolates.

Youil R. et al., Genomics, 1996, Vol. 32, pages 431-435, describes detection of 81 of 81 known mouse beta-globin promoter mutations with T3 endonuclease.

Youil R. et al., PNAS Vol. 92, 1995, pages 87-91, describes screening for mutations by enxyme mis-match cleavage with T4 endonuclease VII.

Oleykowski C.A. et al., Nucleic Acids Research (1998) Vol. 26, pages 4597-4602, describes mutation detection using a novel plant endonuclease.

Condie A. et al., Human Mutation (1993), Vol. 2, pages 58-66, describes detection of point mutations in the p53 gene by single-strand conformation polymorphism, constant denaturant gel electrophoresis, and hydroxylamine and osmium tetroxide techniques.

Accordingly, a first aspect of the present invention provides a set of nucleic acid probes for use in an assay for detecting multi-drug resistant *Mycobacterium sp.* in a sample, which set includes probe 1 comprising a nucleic acid sequence of 10 nucleotides that binds to a first target sequence ACCAGCGGCA, or to the complement thereof; probe 2 comprising a nucleic acid sequence of 10 nucleotides that binds to a second target sequence GCCGGTGGTG, or to the complement thereof; probe 3 comprising a nucleic acid sequence of 10 nucleotides that binds to a third target sequence TATCGTCTCG, or to the complement thereof; probe 4 comprising a nucleic acid sequence of 10 nucleotides that binds to a fourth target sequence TATCATCTCG, or to the complement thereof; probe 5 comprising a nucleic acid sequence of 10 nucleotides that binds to a fifth target sequence GAATTGGCTC, or to the complement thereof; probe 6 comprising a nucleic acid sequence of 10 nucleotides that binds to a sixth target sequence CTGGTCCATG, or to the complement thereof; probe 7 comprising a nucleic acid sequence of 10 nucleotides that binds to a seventh target sequence GGTTGTTCTG, or to the complement thereof; probe 8 comprising a nucleic acid sequence of 10 nucleotides that binds to an eighth target sequence CCCGACAGCG, or to the complement thereof; probe 9 comprising a nucleic acid sequence of 10 nucleotides that binds to a ninth target sequence GCTTGTGGGT, or to the complement thereof; probe 10 comprising a nucleic acid sequence of 10 nucleotides that binds to a tenth target sequence CCAGTGCCGA, or to the complement thereof; wherein each of said probes is 18-25 nucleotides long and wherein, in use once a probe is bound to a respective target sequence, a detectable signal is provided.

It is preferred that each probe comprises a nucleic acid sequence of 15 nucleotides. Thus, in one embodiment, probe 1 comprises a nucleic acid sequence of 15 nucleotides that binds to a first target sequence ATCACCAGCGGCATC, or to the complement thereof; probe 2 comprises a nucleic acid sequence of 15 nucleotides that binds to a second target sequence GATGCCGGTGGTGTA, or to the complement thereof; probe 3 comprises a nucleic acid sequence of 15 nucleotides that binds to a third target sequence ACCTATCGTCTCGCC, or to the complement thereof; probe 4 comprises a nucleic acid sequence of 15 nucleotides that binds to a fourth target sequence ACCTATCATCTCGCC, or to the complement thereof; probe 5 comprises a nucleic acid sequence of 15 nucleotides that binds to a fifth target sequence ATGAATTGGCTCAGC, or to the complement thereof; probe 6 comprises a nucleic acid sequence of 15 nucleotides that binds to a sixth target sequence GTTCTGGTCCATGAA, or to the complement thereof; probe 7 comprises a nucleic acid sequence of 15 nucleotides that binds to a seventh target sequence GCGGGTTGTTCTGGT, or to the complement thereof; probe 8 comprises a nucleic acid sequence of 15 nucleotides that binds to an eighth target sequence AACCCCGACAGCGGG, or to the complement thereof; probe 9 comprises a nucleic acid sequence of 15 nucleotides that binds to a ninth target sequence GGCGCTTGTGGGTCA, or to the complement thereof; probe 10 comprises a nucleic acid sequence of 15 nucleotides that binds to a tenth target sequence GCCCCAGTGCCGACA, or to the complement thereof.

The present invention thus relates to the use of a carefully selected set of probes that enable mutations in three target genes (*katG, inhA* and *rpoB*) to be detected simultaneously in a single assay. Partial gene sequences for the wild-type version of each of these genes are provided as Genbank accession NOs MTU06270, MTU66801 and Z95972, respectively.

Of the set of probes provided by the present invention, probes 1 and 2 target the first gene for INH resistance *(katG),* probes 3 and 4 target the second gene for isoniazid resistance *(inhA),* and probes 5-10 form a scanning array for an 81 base pair region within *rpoB* associated with RIF resistance. The probes of the present invention have been optimised both individually and as a group - each being highly specific, enabling individual base mutations to be detected.

The probes of the present invention have been carefully designed to bind to the target gene sequence based on a selection of desired parameters. It is preferred that the binding conditions are such that a high level of specificity is provided - ie. binding occurs under "stringent conditions". In general, stringent conditions are selected to be about 5°C-lower than the thermal melting point (Tₘ) for the specific sequence at a defined ionic strength and pH. The Tₘ is the temperature (under defined ionic strength and pH) at which 50% of the target sequence binds to a perfectly matched probe. In this regard, the Tₘ of each probe of the present invention, at a salt concentration of about 0.02M or less at pH 7, is preferably above 60°C, more preferably about 70°C. Premixed binding solutions are available (eg. EXPRESSHYB Hybridisation Solution from CLONTECH Laboratories, Inc.), and binding can be performed according to the manufacturer=s instructions. Alternatively, one of a skill in the art can devise variations of these binding conditions.

Following binding, the nucleic acid molecules can be washed to remove unbound nucleic acid molecules, under stringent (preferably highly stringent) conditions. Typical stringent washing conditions include washing in a solution of 0.5-2x SSC with 0.1 % SDS at 55-65°C. Typical highly stringent washing conditions include washing in a solution of 0.1-0.2x SSC with 0.1%SDS at 55-65°C. A skilled person can readily devise equivalent conditions for example, by substituting SSPE for the SSC in the wash solution.

It is preferable to screen the probes to minimise self-complementarity and dimer formation (probe-probe binding). Preferred probes of the present invention are selected so as to have minimal homology with human DNA. The selection process may involve comparing a candidate probe sequence with human DNA and rejecting the probe if the homology is greater than 50%. The aim of this selection process is to reduce annealing of probe to contaminating human DNA sequences and hence allow improved specificity of the assay.

In one embodiment, the present invention provides a set of probes, which set includes probe 1 comprising the sequence TGCCGCTGGT, or a sequence having at least 90% sequence identity thereto; probe 2 comprising the sequence CACCACCGGC, or a sequence having at least 90% sequence identity thereto; probe 3 comprising the sequence CGAGACGATA, or a sequence having at least 90% sequence identity thereto; probe 4 comprising the sequence CGAGATGATA, or a sequence having at least 90% sequence identity thereto; probe 5 comprising the sequence GAGCCAATTC, or a sequence having at least 90% sequence identity thereto; probe 6 comprising the sequence CATGGACCAG, or a sequence having at least 90% sequence identity thereto; probe 7 comprising the sequence CAGAACAACC, or a sequence having at least 90% sequence identity thereto; probe 8 comprising the sequence CGCTGTCGGG, or a sequence having at least 90% sequence identity thereto; probe 9 comprising the sequence ACCCACAAGC, or a sequence having at least 90% sequence identity thereto; probe 10 comprising the sequence TCGGCACTGG, or a sequence having at least 90% sequence identity thereto; wherein the underlined nucleotides within the sequences of probes 1, 2,3 and 4 are essential, and may not be substituted by any other nucleotide, and wherein each of said probes is 18-25 nucleotides long.

In a preferred embodiment, the present invention provides a set of 10 probes, which set includes probe 1 comprising the sequence GATGCCGCTGGTGAT, or a sequence having at least 90% sequence identity thereto; probe 2 comprising the sequence ATCACCACCGGCATC, or a sequence having at least 90% sequence identity thereto; probe 3 comprising the sequence GGCGAGACGATAGGT, or a sequence having at least 90% sequence identity thereto; probe 4 comprising the sequence GGCGAGATGATAGGT, or a sequence having at least 90% sequence identity thereto; probe 5 comprising the sequence AGCTGAGCCAATTCATG, or a sequence having at least 90% sequence identity thereto; probe 6 comprises the sequence AATTCATGGACCAGAACA, or a sequence having at least 90% sequence identity thereto; probe 7 comprising the sequence ACCAGAACAACCCGC, or a sequence having at least 90% sequence identity thereto; probe 8 comprising the sequence ACCCGCTGTCGGGGTT, or a sequence having at least 90% sequence identity thereto; probe 9 comprising the sequence TGACCCACAAGCGCC, or a sequence having at least 90% sequence identity thereto; probe 10 comprising the sequence CTGTCGGCACTGGGGCC, or a sequence having at least 90% sequence identity thereto; wherein the underlined nucleotides within the sequences of probes 1, 2,3 and 4 are essential, and may not be substituted by any other nucleotide, and wherein each of said probes is 18-25 nucleotides long.

Particularly good results have been obtained using a set of 10 probes comprising one of each of probe SEQ ID NOs 1-10 (see Table 1 below).

The present invention thus provides, in a preferred embodiment, a set of probes, which set includes probe 1 comprising the sequence SEQ ID NO 1, or a sequence having at least 90% sequence identity thereto; probe 2 comprising the sequence SEQ ID NO 2, or a sequence having at least 90% sequence identity thereto; probe 3 comprising the sequence SEQ ID NO 3, or a sequence having at least 90% sequence identity thereto; probe 4 comprising the sequence SEQ ID NO 4, or a sequence having at least 90% sequence identity thereto; probe 5 comprising the sequence SEQ ID NO 5, or a sequence having at least 90% sequence identity thereto; probe 6 comprising the sequence SEQ ID NO 6, or a sequence having at least 90% sequence identity thereto; probe 7 comprising the sequence SEQ ID NO 7, or a sequence having at least 90% sequence identity thereto; probe 8 comprising the sequence SEQ ID NO 8, or a sequence having at least 90% sequence identity thereto; probe 9 comprising the sequence SEQ ID NO 9, or a sequence having at least 90% sequence identity thereto; probe 10 comprising the sequence SEQ ID NO 10, or a sequence having at least 90% sequence identity thereto;
wherein nucleotide residue 11 within SEQ ID NOs 1 and 2 is essential and may not be substituted by any other nucleotide;
and wherein nucleotide residue 8 within SEQ ID NOs 3 and 4 is essential and may not be substituted by and other nucleotide, and wherein each of said probes is 18-25 nucleotides long,

**Table 1**

| SEQ. ID. NO. | PROBE NUMBER | PROBE NAME | SEQUENCE OF PROBE |
|---|---|---|---|
| 1 | 1 | K315WTC10T | ctc gat gcc gct ggt gat cgc |
| 2 | 2 | K315GC10T | gcg atc acc acc ggc atc gag |
| 3 | 3 | tomiwt10T | ggc gag acg ata ggt tgt cgg |
| 4 | 4 | tomimut110T | ggc gag atg ata ggt tgt cgg |
| 5 | 5 | MRURP3 | gcc agc tga gcc aat tca tgg ac |
| 6 | 6 | MRURP615T | gcc aat tca tgg acc aga aca acc |
| 7 | 7 | MRURP9 | tgg acc aga aca acc cgc tgt c |
| 8 | 8 | MRURP12 | aca acc cgc tgt cgg ggt tga c |
| 9 | 9 | MRURP17 | ggt tga ccc aca agc gcc gac |
| 10 | 10 | MRU1371A | cga ctg tcg gca ctg ggg ccc gg |

Where sequences having "at leat 90% sequence identity" to a sequence of the present invention are referred to in the present description, the present invention also embraces probe sequences that have preferably at least 95% sequence identity, more preferably at least 98% sequence identity, most preferably at least 99% sequence identity to probe sequences of the present invention.

Probe sequences having at least 90% sequence identity, preferably at least 95% sequence identity, more preferably at least 98% sequence identity, most preferably at least 99% sequence identity to probe sequences of the present invention may be identified by sequence alignments using conventional software, for example the Bioedit™ package, available free online, and the Sequencher™ package, provided by Sequencher Gene Codes Corporation, 640 Avis Drive Suite 310, Ann Arbor MI 48108.

An alternative means for defining probe sequences that are homologous to probe sequences of the present invention is by defining the number of nucleotides that differ between the homologous sequence and the sequence of the invention. In this regard, the present invention embraces probe sequences that differ from the probe sequences of the invention by no more than 5 nucleotides, preferably by no more than 4 nucleotides, more preferably by no more than 3 nucleotides, yet more preferably by no more than 2 nucleotides, and most preferably by no more than 1 nucleotide. The underlined nucleotides in the sequences of probes 1, 2, 3 and 4, must not however, be substituted by any other nucleotide.

In the present invention, a "complement" or "complementary strand" means the non-coding (anti-sense) nucleic acid strand, which may bind via complementary base-pairing to a coding strand. Hence, the present invention also embraces use of the complements of the probes described herein. By way of example, the complement of Probe 1, above, has the sequence GAG CTA CGG CGA CCA CTA GCG and the complement of probe 2, above, has the sequence CGC TAG TGG TGG CCG TAG CTC. It is well known in the art to work out the sequence of a complementary strand by using the complementary base-pairing rules, if the sequence of the coding strand is known.

In one embodiment of the present invention, the probes may be immobilised onto a solid support or platform: The support may be a rigid solid support made from, for example, glass or plastic, or else the support may be a nylon or nitrocellulose membrane, or other membrane. 3D matrices are suitable supports for use with the present invention - eg. polyacrylamide or PEG gels. In one embodiment, the solid support may be in the form of beads, which may be sorted by size or fluorophores.

The probes may be immobilised to the solid support by a variety of means. By way of example, probes may be immobilised onto a nylon membrane by UV cross-linking. Biotin-labelled probes may be bound to streptavidin-coated substrates, and probes prepared with amino linkers may be immobilised onto silanised surfaces. Another means of immobilising probe is via a poly-T tail, preferably at the 3' end. The poly-T tail consists of a run of from 1 to 100 thymine residues added to the probe at the 3' end with a terminal transferase. Preferably, from 1 to 20 thymine residues are added. The poly-T tail is then baked or UV cross-linked onto the solid substrate: Addition of a poly-T tail appears to have two functions. First, the poly-T tail increases the amount of probe that is immobilised onto the solid support. Second, the poly-T tail conforms the probe in such a way as to improve the efficiency of hybridisation. Once a probe of the present invention is bound to a target *Mycobacterium sp.* (eg. *M. tuberculosis)* nucleic acid, a detectable signal is provided that may be detected by known means. A detectable signal may be a radioactive signal but is preferably a fluorescent signal (most preferably a change in fluorescence), or a chromogenic signal employing biotin or igoxygenin.

The present invention also provides a method of detecting multi-drug resistant *Mycobacterium sp.* In a sample, in particular, members of the MTC such as *M. tuberculosis.* The method comprises contacting a set of probes according to the present invention with a nucleic acid-containing sample, wherein, once a probe is bound to a target *Mycobacterium sp.* Nucleic acid in the sample, a detectable signal is provided; and detecting said detectable signal.

A sample may be for instance, a food, sewerage or clinical sample. A particular application of the method is for detection of *Mycobacterium sp.* in a clinical sample. Clinical samples may include broncho-alveolar lavage specimens (BALS), induced sputa, oropharyngeal washes, blood or other body fluid samples.

In the present method, it is preferred that the presence of multi-drug resistant *Mycobacterium sp.* in said sample is confirmed by detecting a detectable signal provided by probes 2 and 4 and their respective bound target *Mycobacterium sp.* nucleic acid sequences in the sample; and detecting the absence of a detectable signal provided by probes 1, 3, 5, 6, 7, 8, 9 and 10 and their respective target *Mycobacterium sp.* nucleic acid sequences.

It is preferred that the present method allows confirmation of the absence of multidrug resistant *Mycobacterium sp.* from said sample by detecting a detectable signal provided by probes 1, 3, 5, 6, 7, 8, 9 and 10 and their respective bound target *Mycobacterium sp.* nucleic acid sequence in the sample; and detecting the absence of a detectable signal provided by probes 2 and 4 and their respective target *Mycobacterium sp.* nucleic acid sequences.

In this regard, probes 2 and 4 bind to mutant *M. tuberculosis* nucleic acid. Probe 2 binds to a specific mutated target site within the *katG* gene, and probe 4 binds to a specific mutated target site within the *inhA* gene. Probes 2 and 4 only bind to their specific mutated sequence, and do not bind the wild-type target sequence. Binding of probe 2 and/ or 4 to *M. tuberculosis* nucleic acid in the sample therefore indicates that the sample contains nucleic acid having a mutation in *katG* and/or *inhA* respectively. The mutations that are detected by probes 2 and 4 are involved in resistance to isoniazid.

Probes 1, 3 and 5-10 bind to wild type *M. tuberculosis* nucleic acid. Probe 1 binds to a target site within the *katG* gene - the wild-type version of the sequence bound by probe 2. Probe 3 binds to a target site within the *inh*A gene - the wild-type version of the sequence bound by probe 4. Hence, binding of probe 1 and/or 3 to nucleic acid in the sample indicates that the sample contains nucleic acid that is wild-type for *katG* and/or *inhA* respectively. Probes 1 and 3 only bind the wild-type sequence, and do not bind when their target sequence is mutated.

Probes 5-10 bind only to wild-type *M. tuberculosis* nucleic acid, within an 81 base pair target region of the wild-type *rpoB* gene. If the sample contains wild-type *rpoB* nucleic acid, then all of probes 5-10 will bind. On the other hand, if the nucleic acid in the sample has specific *rpoB* mutations associated with rifampin resistance, then fewer than 6 *rpoB* probes will bind.

In more detail, the presence of multi-drug resistant *Mycobacterium sp.* nucleic acid is confirmed in the sample by detecting binding of at least one of probes 2 and 4 to mutant nucleic acid (ie. detecting a detectable signal provided by probe 2 and/ or 4 and their respective bound target sequences), and detecting non-binding of at least one of probes 1, 3 and at least one of probes 5-10 to wild-type nucleic acid (ie. detecting absence of a detectable signal provided by probes 1, 3 and 5-10 and their respective target sequences).

On the other hand, if probes 2 and 4 do not bind to nucleic acid in the sample (as evidenced by the absence of a detectable signal provided by probes 2 and 4 and their respective target sequences), but both of probes 1, 3 and all of probes 5-10 do bind to nucleic acid in the sample (as evidenced by detection of a detectable signal provided by probes 1, 3 and 5-10 and their respective bound target sequences), this indicates that the sample does not contain multi-drug resistant *Mycobacterium tuberculosis* nucleic acid.

If a detectable signal is provided by all of the probes and their respective target nucleic acid sequences, this indicates the presence in the sample of *Mycobacterium tuberculosis* nucleic acid from bacteria that have a wild-type *rpoB* gene - ie. sensitive to rifampin. This sample also contains nucleic acid from *Mycobacterium tuberculosis* that have mutant *katG* and *inhA* genes - ie. resistant to isoniazid, as well as nucleic acid from *Mycobacterium tuberculosis* that have wild-type *katG* and *inhA* genes - ie. sensitive to isoniazid. Hence, a mixture of INH mono-resistant *Mycobacterium tuberculosis* and wild-type (RIF/ INH sensitive) *Mycobacterium tuberculosis* are detected.

It is an option to use more than 10 probes - ie. to include further probes in addition to the 10 probes described in detail herein. The further probes may be useful for detection of mutations in other *Mycobacterium sp.* genes, or for detection of nucleic acids other than *Mycobacterium sp.* nucleic acid, for example, as part of a wider diagnostic array for analysis of other bacterial species.

In one embodiment, one or more of probes 5-10 described in detail above (SEQ ID NOs: 5-10) may be substituted or used in combination with one or more, preferably 2, 3, 4, 5, 6, 7, 8, 9, 10, 11,12,13,14,15,16, 17, 18, 19, 20, 21 or 22 of the probes provided in Table 1a below (probes 11-32, SEQ ID NOs: 11-32). Each of probes 11-32 binds to wild-type *M. tuberculosis* nucleic acid, within the wild-type *rpoB* gene. Hence, if the sample contains wild-type *rpoB* nucleic acid, then probes 11-32 will bind to nucleic acid in the sample, and if the sample contains mutant *rpoB* nucleic acid, then at least one of probes 11-32 will not bind to nucleic acid in the sample. Thus, probes 11-32 are useful for detecting *M. tuberculosis* that are resistant to rifampin.

**Table 1a**

| SEQ ID NO | PROBE NUMBER | PROBE NAME | SEQUENCE OF PROBE |
|---|---|---|---|
| 11 | 11 | MRURP1 | cag cca gcc agc tga gcc aat tc |
| 12 | 12 | MRURP2 | cca gcc agc tga gcc aat tca tg |
| 13 | 13 | MRURP4 | agc tga gcc aat tca tgg acc ag |
| 14 | 14 | MRURP5⁻ | tga gcc aat tca tgg acc aga aca |
| 15 | 15 | MRURP7 | aat tca tgg acc aga aca acc cgc |
| 16 | 16 | MRURP8 | tca tgg acc aga aca acc cgc tg |
| 17 | 17 | MRURP10 | acc aga aca acc cgc tgt cgg g |
| 18 | 18 | MRURP11 | aga aca acc cgc tgt cgg ggt t |
| 19 | 19 | MRURP13 | acc cgc tgt cgg ggt tga ccc |
| 20 | 20 | MRURP14 | cgc tgt cgg ggt tga ccc aca a |
| 21 | 21 | MRURP15 | tgt cgg ggt tga ccc aca agc g |
| 22 | 22 | MRURP16 | cgg ggt tga ccc aca agc gcc |
| 23 | 23 | MRURP18 | tga ccc aca agc gcc gac tgt c |
| 24 | 24 | MRURP19 | ccc aca agc gcc gac tgt cgg |
| 25 | 25 | MRURP20 | aca agc gcc gac tgt cgg cgc |
| 26 | 26 | MRURP21 | agc gcc gac tgt cgg cgc tgg |
| 27 | 27 | MRURP22 | gcc gac tgt cgg cgc tgg ggc |
| 28 | 28 | MRURP23 | gac tgt cgg cgc tgg ggc ccg |
| 29 | 29 | MRUR24 | tgt cgg cgc tgg ggc ccg gc |
| 30 | 30 | MRURP25 | cgg cgc tgg ggc ccg gcg gt |
| 31 | 31 | MRURP26 | cgc tgg ggc ccg gcg gtc tg |
| 32 | 32 | MRURP27 | tgg ggc ccg gcg gtc tgt cac |

The present invention also embraces probe sequences that have at least 90% sequence identity, preferably at least 95% sequence identity, more preferably at least 98% sequence identity, most preferably at least 99% sequence identity to probe sequences 11-32 of the present invention.

The present assay may be used with or without a prior amplification step, depending on the concentration of *M. tuberculosis* nucleic acid that is available. Amplification may be carried out by methods known in the art, preferably by PCR.

Preferably, the step of amplifying *Mycobacterium sp.* nucleic acid in the nucleic acid-containing sample is carried out prior to detection of signal. Most preferably, the step of amplifying *Mycobacterium sp.* nucleic acid in the sample is carried out prior to contacting the set of probes with the nucleic acid-containing sample.

Amplification of *M. tuberculosis* nucleic acid is preferably carried out using a pair of sequence specific primers, which bind to a target site within the *M. tuberculosis* nucleic acid and are extended, resulting in nucleic acid synthesis. Primers designed to bind to the target gene sequence based on the selection of desired parameters, using conventional software, such as Primer Express (Applied Biosystems). In this regard, it is preferred that the binding conditions are such that a high level of specificity is provided. The melting temperature (Tₘ) of the primers is preferably 50°C or higher, and most preferably about 60°C. The primers are preferably screened to minimise self-complementarity and dimer formation (primer-to-primer binding).

The primer pair comprises forward and reverse oligonucleotide primers. A forward primer is one that binds to the complementary, non-coding (anti-sense) strand of the target *M. tuberculosis* nucleic acid and a reverse primer is one that binds to the coding (sense) strand of the target *M. tuberculosis* nucleic acid.

The forward and reverse oligonucleotide primers are typically 1 to 50 nucleotides long, preferably 10 to 40 nucleotides long, more preferably 15-25 nucleotides long. It is generally advantageous to use short primers, as this enables faster annealing to target nucleic acid.

Particularly good results have been obtained using the forward (F) and reverse (R) oligonucleotide primers shown in Table 2 below.

**Table 2**

| SEQ ID NO. | Gene | Primer Name | Primer Sequence | F or R |
|---|---|---|---|---|
| 33 | katG | KatGP5BIO | CGCTGGAGCAGATGGGCTTGG | F |
| 34 | katG | KatGP6BIO | GTCAGCTCCCACTCGTAGCCG | R |
| 35 | inhA | INHAP3BIO | GCAGCCACGTTACGCTCGTGG | F |
| 36 | inhA | TOMIP2BIO | CGATCCCCCGGTTTCCTCCGG | R |
| 37 | rpoB | FTIP1BIO | GGTCGGCATGTCGCGGATGG | F |
| 38 | rpoB | BrpoB1420R | GTAGTGCGACGGGTGCACGTC | R |

It will, however, be appreciated that variants may be employed, which differ from the above-mentioned primer sequences by one or more nucleotides. In this regard, conservative substitutions are preferred. It is also preferred that primers do not differ from the above-mentioned primers at more than 5 nucleotide positions.

It is an option for the probe to be labelled, however, it is preferred that the probe is unlabelled and that, instead, the target *Mycobacterium sp.* nucleic acid in the sample is labelled. The target nucleic acid may be labelled during PCR amplification, by using labelled primers. Thus, in one embodiment, the target nucleic acid in the sample is labelled and the assay comprises detecting the label and correlating presence of label with presence of *Mycobacterium sp.* nucleic acid. The label may be a radiolabel but is preferably non-radioactive, such as biotin, digoxygenin or a fluorescence signal such as fluorescein-isothiocyanate (FITC). The label may be detected directly, such as by exposure to photographic or X-ray film, or indirectly, for example, in a two-phase system. An example of indirect label detection is binding of an antibody to the label. In another example, the target nucleic acid is labelled with biotin and is detected using streptavidin bound to a detectable molecule or to an enzyme, which generates a detectable signal. Colorimetric detection systems may also be employed, such as alkaline phosphatase plus NBT/BCIP.

Also referred to is a single probe selected from the group consisting of: probe 5 comprising the sequence GAGCCAATTC, or the complement thereof; or a sequence having at least 90% sequence identity thereto, or the complement thereof; probe 6 comprising the sequence CATGGACCAG, or the complement thereof; or a sequence having at least 90% sequence identity thereto, or the complement thereof; probe 7 comprising the sequence CAGAACAACC, or the complement thereof; or a sequence having at least 90% sequence identity thereto, or the complement thereof; probe 8 comprising the sequence CGCTGTCGGG, or the complement thereof; or a sequence having at least 90% sequence identity thereto, or the complement thereof; probe 9 comprising the sequence ACCCACAAGC, or the complement thereof; or a sequence having at least 90% sequence identity thereto, or the complement thereof; probe 10 comprising the sequence TCGGCACTGG, or the complement thereof; or a sequence having at least 90% sequence identity thereto, or the complement thereof; for use in an assay for detecting multi-drug resistant *Mycobacterium sp.* in a sample.

Where sequences having "at least 90% sequence identity" to a sequence of the present invention are referred to in the present description, probe sequences that have preferably at least 95% sequence identity, more preferably at least 98% sequence identity, most preferably at least 99% sequence identity to probe sequences of the present invention are also referred to.

The present invention also provides use of a single probe according to the present invention for the manufacture of a composition for detecting multi-drug resistant *Mycobacterium sp.* nucleic acid, preferably multi-drug resistant MTC nucleic acid, such as multi-drug resistant *M. tuberculosis* nucleic acid, in a sample.

Also provided by the present invention is a kit for detection of multi-drug resistant *Mycobacterium sp.* nucleic acid, preferably multi-drug resistant MTC nucleic acid, such as multi-drug resistant *M. tuberculosis* nucleic acid, comprising a set of probes according to the present invention.

In accordance with an alternative aspect of the present invention, there is provided an alternative set of nucleic acid probes for use in an assay for detecting multi-drug resistant *Mycobacterium sp.* in a sample, which set includes probe 1 comprising a nucleic acid sequence of 10 nucleotides that binds to a first target sequence ACCAGCGGCA, or to the complement thereof; probe 2 comprising a nucleic acid sequence of 10 nucleotides that binds to a second target sequence GCCGGTGGTG, or to the complement thereof; probe 5 comprising a nucleic acid sequence of 10 nucleotides that binds to a fifth target sequence GAATTGGCTC, or to the complement thereof; probe 6 comprising a nucleic acid sequence of 10 nucleotides that binds to a sixth target sequence CTGGTCCATG, or to the complement thereof; probe 7 comprising a nucleic acid sequence of 10 nucleotides that binds to a seventh target sequence GGTTGTTCTG, or to the complement thereof; probe 8 comprising a nucleic acid sequence of 10 nucleotides that binds to an eighth target sequence CCCGACAGCG, or to the complement thereof; probe 9 comprising a nucleic acid sequence of 10 nucleotides that binds to a ninth target sequence GCTTGTGGGT, or to the complement thereof; probe 10 comprising a nucleic acid sequence of 10 nucleotides that binds to a tenth target sequence CCAGTGCCGA, or to the complement thereof; and wherein, in use, once a probe is bound to a respective target sequence, a detectable signal is provided, and wherein each of said probes is 18-25 nucleotides long

This alternative set of probes differs from the set of probes described earlier in that probes 3 and 4 (which target the *inhA* gene) are not essential. However, this set of probes includes probes 1 and 2, which target the *katG* gene, and probes 5-10, which target the *rpoB* gene. Hence, this set of probes is useful for detecting mutations in the *rpoB* gene (conferring RIF resistance) and the *katG* gene (conferring INH resistance).

Preferably, this alternative set of probes includes probe 1 comprising the sequence TGCCGCTGGT, or a sequence having at least 90% sequence identity thereto; probe 2 comprising the sequence CACCACCGGC, or a sequence having at least 90% sequence identity thereto; probe 5 comprising the sequence GAGCCAATTC, or a sequence having at least 90% sequence identity thereto; probe 6 comprising the sequence CATGGACCAG, or a sequence having at least 90% sequence identity thereto; probe 7 comprising the sequence CAGAACAACC, or a sequence having at least 90% sequence identity thereto; probe 8 comprising the sequence CGCTGTCGGG, or a sequence having at least 90% sequence identity thereto; probe 9 comprising the sequence ACCCACAAGC, or a sequence having at least 90% sequence identity thereto; probe 10 comprising the sequence TCGGCACTGG, or a sequence having at least 90% sequence identity thereto; wherein the underlined nucleotides within the sequences of probes 1, 2,3 and 4 are essential, and may not be substituted by any other nucleotide.

In accordance with this alternative aspect of the invention, there is also provided a method of detecting the presence or absence of multi-drug resistant *Mycobacterium sp*. in a sample, comprising: (a) contacting the alternative set of probes as described above with a nucleic acid-containing sample wherein, once a probe is bound to *Mycobacterium sp.* nucleic acid in the sample, a detectable signal is provided; and (b) detecting said detectable signal. Thus, this method allows mutations in the *katG* and *rpoB* genes to be detected simultaneously in a single assay.

The present invention also provides a kit for detection of multi-drug resistant *Mycobacterium sp.* nucleic acid comprising the alternative set of probes as described above. Using this kit, mutations in the *rpoB* and *katG* genes may be detected simultaneously in a single assay.

The embodiments of the invention are discussed in more detail by means of the Examples described below. The results referred to in the Examples are illustrated by the accompanying drawings, in which:
Fig.1 shows the design of individual macroarrays according to the present invention.
Fig. 2 shows the appearance of developed macroarray membranes.
Fig. 3 shows the spectrum of mutations involved in RIF and INH resistance identified in the Samara isolates.
Fig. 4A shows a schematic of the MDR-screen macroarray;
Fig. 4B shows patterns generated by the *M. tuberculosis* strains.

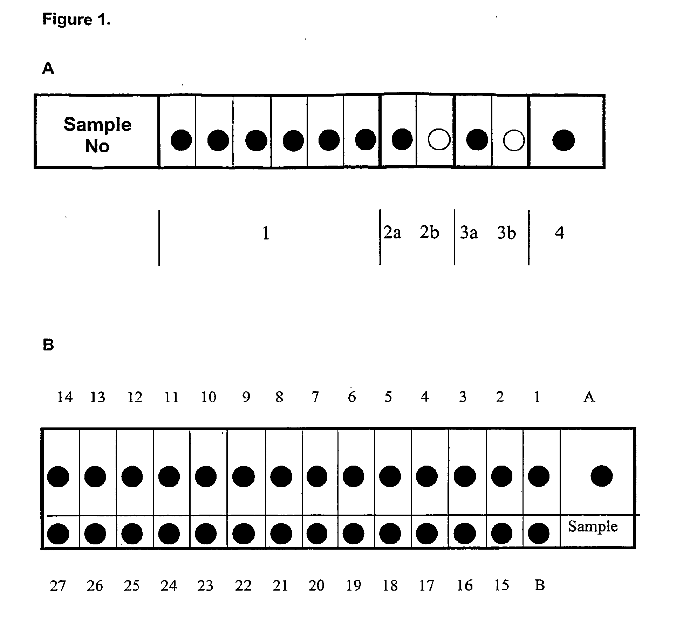

In more detail, Figure 1A illustrates a screening macroarray comprising 6 different non-mutant (wild-type) *rpoB* gene probes (1), non-mutant *katG* probe (2a), mutant (codon 315) *katG* gene probe (2b), non-mutant *inhA* gene probe (3a), mutant (codon 280) *inhA* gene probe (3b) and a colour development control probe (4).

Figure 1B illustrates a scanning macroarray, comprising non-mutant (wild-type) *rpoB* probes (1-27), a colour development control probe (B) and an ink spot (A) for orientation.

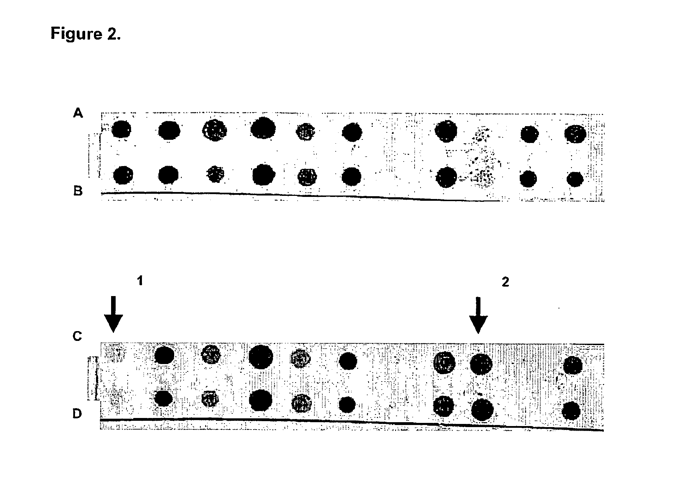

Figure 2A,B illustrates the appearance of a developed macroarray membrane that has been contacted with *Mycobacterium tuberculosis* wild-type isolates having no mutations in the *rpoB, katG* or *inhA* genes (ie. RIF/INH sensitive isolates). Probes 1, 3, and 5-10 have all bound nucleic acid in the sample, indicating the presence of a RIF/ INH sensitive genotype.

Figure 2C,D illustrates the appearance of a developed macroarray membrane that has been contacted with *Mycobacterium tuberculosis* isolates having mutations in codon 531 of *rpoB* (1) and codon 315 of *katG* (2). Fewer than 6 of the *rpoB* probes are bound, indicating detection of the *rpoB* mutation, and the mutant *katG* probe is bound, indicating detection of the *katG* mutation - thus indicating the presence of a RIF/INH resistant (ie. multi-drug resistant) genotype.

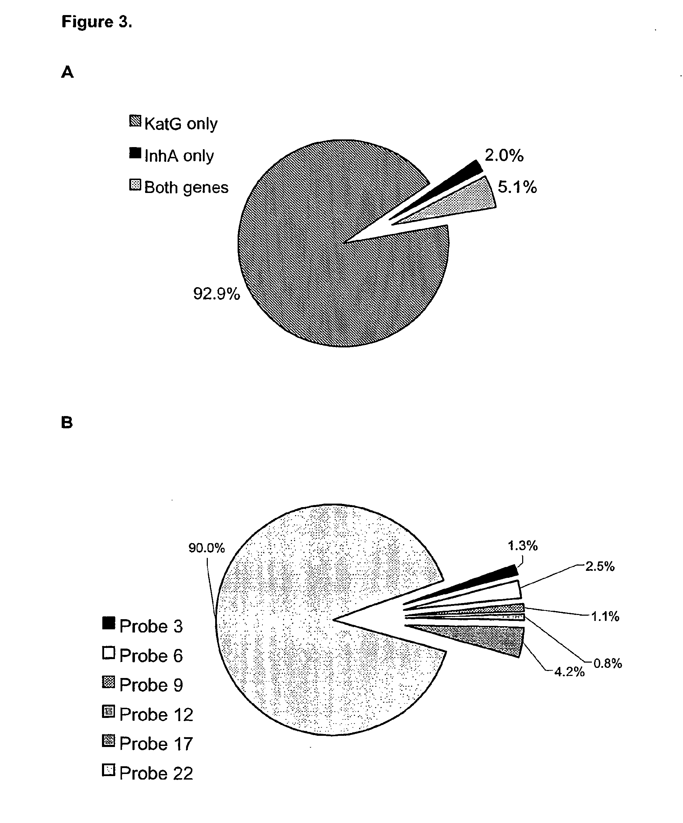

Figure 3A illustrates the mutations associated with INH resistance in the Samara isolates. 92.9% of mutations are in *katG* only, with 2.0% of mutations in *inhA* only, and 5.1% of mutations in both genes.

Figure 3B illustrates the mutations associated with RIF resistance in the Samara isolates. Probe 3 of the scanning array detected 1.3% of mutations, probe 6 detected 2.5% of mutations, probe 9 detected 1.1 % of mutations, probe 12 detected 0.8% of mutations, probe 17 detected 4.2% of mutations and probe 22 detected 90.0% of mutations.

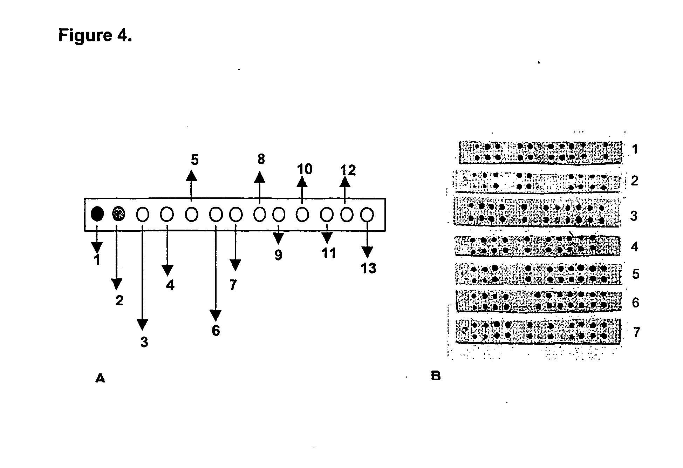

Figure 4A illustrates a macroarray according to the present invention, as used in Example 4 (below). The key to the spots is as follows:
- 1: ink spot
- 2: control
- 3: probe MtbC for detecting probe an *M. tuberculosis* complex specific locus of *M. tuberculosis rpoB*
- 4: probe 1 (of Table 1 above)
- 5: probe 2 (of Table 1 above)
- 6: probe 3 (of Table 1 above)
- 7: probe 4 (of Table 1 above)
- 8: probe 5 (of Table 1 above)
- 9: probe 6 (of Table 1 above)
- 10: probe 7 (of Table 1 above)
- 11: probe 8 (of Table 1 above)
- 12: probe 9 (of Table 1 above)
- 13: probe 10 (of Table 1 above)

Figure 4B illustrates patterns obtained for the different *M. tuberculosis* strains:
- pattern 1: strain with the *kat*G315 AGC-ACC mutation + *rpo*B526 mutant allele;
- pattern 2: strain with the *kat*G315 AGC-ACC mutation + insertion in the *rpo*B gene (ins TTC at the 514 codon);
- pattern 3: wild type strain;
- pattern 4: strain with the *rpo*B531 mutant allele;
- pattern 5: strain with the *kat*G315 AGC-ACA mutation;
- pattern 6: strain with the *inh*A^{C-15T} mutation in the regulatory region of the *mab*A*- inh*A operon;
- pattern 7: strain with the *rpo*B516 mutant allele.

### Examples

### Example 1 - Amplification of Mycobacterium tuberculosis nucleic acid.

A total of 234 clinical isolates of *Mycobacterium tuberculosis* from Samara (Central Russia) were available for both phenotypic and genotypic testing.

All sputum specimens where cultured on Lowenstein-Jensen media, and their identity was confirmed using a combination of growth; macroscopic and microscopic appearance; and DNA hybridisation tests. Drug susceptibility testing was performed using the known resistance ratio method.

DNA was extracted by heating cell suspensions with an equal volume of chloroform at +80°C for 30 minutes, followed by cooling on ice and centrifugation. The upper phase (crude cell lysate) was used for PCR amplification.

Multiplex PCR amplification of regions of the *rpoB, katG* and *inhA* genes was carried out using three pairs of primers, followed by dot-hybridization of the amplification products with normal and mutant oligonucleotide probes (single-stranded fragments of *rpoB, katG* and *inhA* genes in which mutations occur) immobilized on nylon membrane strips (Osmonics Inc., USA).

In more detail, biotin-labelled PCR products were generated in a multiplex PCR using three pairs of primers. The first pair was for amplification of a fragment including the 81 bp "core" region of the *rpoB* gene, for detection of mutations consistent with RIF resistance. The second pair was for amplification of a *katG* gene fragment, including codon 315. The third pair was for the amplification of a *inhA* gene fragment, including the regulatory region.

PCR was conducted in a 20 µl volume, containing 2 µl 10x PCR buffer (Bioline Ltd., London UK); 0.5 unit Taq-polymerase (Bioline); 0,5 µl 2mM dNTP mixture (Bioline); 20µM each of six primers and 1 µl of a DNA extract prepared as described above. Thermal cycling was performed on a Perkin Elmer 9700 Thermocycler using the following amplification programme parameters:
Hold 5 min 95°C
15 sec 95°C
30 cycles 30 sec 65°C
60 sec 72°C
Hold 5 min 72°C

The presence of PCR products (3 fragments of 260 bp; 150 bp; 140 bp in length) was detected by electrophoresis in 2.0% agarose gels stained with ethidium bromide. The PCR products were then available for hybridization, with a streptavidin-alkaline phosphatase colour development system being used to visualise the results.

### Example 2 - Hybridisation

The membranes were spotted with oligonucleotide probes using a spotting device (BioGene, UK) in a specific order to produce the arrays (see Figure 1). After UV cross-linking and washing twice in 0.5x SSC, the membranes were air-dried and cut into separate strips, and placed into 2ml plastic tubes.

The first array was used to screen the isolates and comprised probes to detect the most frequent mutations in *rpoB, katG* and *inhA* genes (see Figure 1A). For the *rpoB* gene, the array included 6 non-mutant probes (probe 3 to detect mutations in codons 511, 513 and 514; probes 6 and 9 for codons 513, 514 and 516; probe 12 for codon 516; probe 17 for codon 526 and probe 22 for codon 531). Probes for wild type and the most frequent mutations in the *katG* gene (AGC→ACC in codon 315) and the regulatory region of the *inhA* gene (G→T in codon 280) were also included.

A second scanning array was developed to detect other less common mutations in the *rpoB* gene. A total of 27 non-mutant oligonucleotide probes for the *rpoB* gene were designed to cover the whole of the 81 bp core region - ie. the sequence in which mutations responsible for rifampin resistance would be found (See Figure 1 B).

Hybridization was performed as follows. Briefly, amplification products were denatured by adding an equal volume of the denaturation solution (0.4M NaOH, 0.02M EDTA) for 15 min at room temperature. In each tube containing an individual array, 500µl of hybridization solution (5xSSPE; 0.5% SDS) and 20µl of denatured PCR products were added. Hybridization was performed in rotating tubes in a hybridization oven at 72°C for 30 min. Membranes were then washed twice in 0.1 M Tris-0.1 M NaCl solution (pH7.5) and incubated for 1 min in 0.1 % Blocking reagent solution (Roche, Mannheim, Germany). After incubation in streptavidin-alkaline phosphatase conjugate solution (1:100) (BioGenex, San Ramon, USA) for 30 min at room temperature and washing, membranes were incubated in 1:250 NBT-BCIP solution (Nitro Blue Tetrasolium, USB, Cleveland, USA, 75mg/ml in DMF, Bromo-Chloro-Indolil Phosphate, USB, Cleveland, USA, 50mg/ml in DMF) in a light-proof container for colour development, washed and air-dried.

### Sequencing

Sequencing of *rpoB* and *katG* gene fragments of selected isolates was performed to verify the results of the macroarray drug susceptibility tests. Template DNA was prepared as described above by chloroform extraction. Amplification products were diluted 1:25 in water and sequenced using a Beckman Coulter SEQ8000 Genetic Analysis System. Sequence data was analysed using SEQ8000 software.

### Results

All Samara cultures (except for one, identified as *M. fortuitum)* were correctly identified as *M. tuberculosis,* using routine phenotypic tests in the reference laboratory.

Multiplex amplification of 233/234 specimens from the Russian mycobacterial cultures was successful (except for the *M. fortuitum*). The appearance of the developed membranes is shown in Figure 2. The results of the macroarray drug susceptibility tests are shown in Table 3 below.

**Table 3**

| Results of rifampin and isoniazid resistance detection using a screening macroarray technique in new cases and chronic cases. | | |
|---|---|---|
| Presence of mutations | New cases (n=78) | Chronic cases (n=156) |
| Mutations consistent with RIF resistance (*rpoB* gene) | 32 (41.0%) | 8 (56.4%) |
| Mutations consistent with INH resistance *(katG* and *inhA* genes) | 45 (57.7%) | 22 (78.2%) |
| Mutations consistent with both RIF and INH resistance | 29 (37.2%) | 87 (55.8%) |

In total, 48.7% of isolates possessed mutations consistent with resistance to rifampin, 67.9% of isolates with resistance to isoniazid, and 46.5% of isolates possessed mutations both in *rpoB* and *katG* (or *inhA*) genes. All except one of the *M. tuberculosis* strains isolated from patients with chronic tuberculosis with mutations consistent with rifampin resistance also possessed mutations consistent with isoniazid resistance.

Results of genotypical (macroarray) and phenotypical drug susceptibility testing were concordant in 90.4% for isoniazid and 79.3% for rifampin resistance. The differences in most cases (in over 60% of cases) were due to phenotypically defined resistance in the absence of mutations associated with resistance identified by the screening macroarray. This suggests that resistance to either RIF or INH may also be associated with other mutations.

We then analysed the spectrum of mutations consistent with resistance to rifampin and isoniazid (see Figure 3). In over 90% of cases, resistance to INH was due to mutations in codon 315 of *katG* gene only. In 2.0% of cases, resistance was due to mutations in the *inhA* gene only, and in 5.1% of cases, both genes contributed to resistance development (see Figure 3A). Of all isolates carrying mutations consistent with RIF resistance, 90.0% possessed mutations in codon 531, and 4.2% in codon 526 of the *rpoB* gene (see Figure 3B). Other mutations (in codons 511-516) were detected in fewer than 6.0% of resistant strains. The second macroarray was designed to detect additional mutations within the 81 bp core region that might be consistent with rifampin resistance in 27 *M. tuberculosis* DNA specimens that had been previously determined as 'wild type' on the screening array (see Table 4).

**Table 4**

| Scanning array results of repeated molecular DST for specimens with disagreements. | | |
|---|---|---|
| Scanning array results | No of discordant results | |
| | Macroarray "wild-type" but phenotypically RIF resistant (n=27) | Macroarray "mutant" but phenotypically RIF sensitive (n=20) |
| Mutant | 20 | 3 |
| Wild-type | 7 | 17 |

Of the 27 *M. tuberculosis* isolates that had been previously suggested to be wild type, twenty specimens (74.7%) had further mutations consistent with rifampin resistance; but seven samples (25.9%) that were phenotypically resistant did not have any mutations in the core region.

To verify the macroarray drug resistance tests results, *rpoB and katG* gene fragments were sequenced in selected isolates. *RpoB* gene sequencing was performed for the seven phenotypically rifampin resistant isolates mentioned above. Two of these isolates were found to possess point mutations in the *rpoB* gene: one has a CAC→CTC (H→Y) substitution in codon 526 and another has a CTG→CCG (L→P) substitution in codon 533. In another five isolates (4.2% of the total number of rifampin resistant strains) no mutations were detected, suggesting that in these cases rifampin resistance was due to mutations in other genes.

Fragments of the *katG* gene were sequenced from 6 resistant and 6 sensitive isolates selected according to the macroarray results. All phenotypically sensitive isolates were found to be wild type and possessed no mutations. In the 6 isolates having mutations in the *katG* gene according to the macroarray, the most common substitution AGC→ACC (S315T) was detected.

### Discussion

Combining results from the two macroarrays enabled detection of mutations consistent with resistance in 95.3% of the cultures that were phenotypically rifampin resistant, and in 90.4% of the cultures that were phenotypically isoniazid resistant. These figures for concordance are higher than previously reported for non-commercial molecular drug susceptibility analysis systems, confirming that the inclusion of additional probes into an array increases system performance. Sequence analysis of the *rpoB* and *katG* genes proved the macroarray method to be highly specific.

### Example 3

### Cultures studied:-

Panel one. 465 INH-resistant strains of *M. tuberculosis,* with or without concurrent RIF resistance, were used to evaluate the multi-drug resistance (MDR) screen array in a retrospective study. These strains represented all INH-resistant strains from January 1998 to December 2003 referred to the HPA Mycobacterial Reference Unit (MRU).

Panel two. 605 consecutive mycobacterial cultures referred to the HPA MRU for identification and drug susceptibility testing-between September and December 2003 were used in a prospective study of the performance of the macroarray.

Mycobacterial cultures were cultured on to Lowenstein-Jensen media or liquid culture media (either MGIT, Becton Dickinson, UK or MB BacT Alert, Biomerieux, Cambridge, UK). Cultures were identified using a combination of microscopic and macroscopic appearance, growth characteristics, biochemical testing and DNA hybridisation (Accuprobe; Genprobe, San Diego, USA). Resistance to isoniazid and rifampicin were determined using the resistance ratio method on Lowenstein-Jensen media (REF).

### DNA extraction

DNA was extracted from mycobacteria using by chloroform extraction. A loop of bacterial culture or 100 µl of liquid culture media was transferred to a microcentrifuge tube and suspended in 100µl purified water, and an equal volume of chloroform was added. The tubes were heated at 80°C for 20 minutes, placed in the freezer for 5 minutes, mixed briefly by vortex and centrifuged for 3 minutes at 12000g just prior to adding to the PCR.

### PCR

Target DNA was amplified by PCR using biotinylated primers at the 5' end to label the PCR products. The reaction mixture of 20µl contained 5µl of purified water, 10 µl of 2x reaction buffer, 5µl) of primer mix, 0.2µl of Taq DNA polymerase (5units/µl, Bioline) and 1 µl of DNA template. The 2x buffer reaction contained 2.0ml of 10x Ammonium reaction buffer (NH₄ Bioline), 600µl of 50mM Magnesium Chloride (MgCl₂, Bioline), 40µl of each 100mM dNTP (dATP, dCTP, dGTP and dTTP, Bioline) and 7240µl of purified water. The primer mix contained 2.5µl of primers katPGBIO and katP6BIO (200µM each), 10µl of primers inhAP, TomiP2BIO, IP1 (de Beenhouwer *et al.,* 1995) and BrpoB1420R (200µM each), and 455µl of purified water.

The amplification reaction was carried out in a DNA thermocycler (GeneAmp PCR System 9700, Applied Biosystems, UK). The thermocycler reaction conditions were 3 min at 95°C, 15 sec at 95°C, 30 sec at 65°C, 60 sec at 72°C for 30 cycles and a final extension cycle of 5 min at 72°C.

### MDR-screen macroarray

The macroarray consisted of 11 probes immobilized as spots on a nylon membrane strip (MagnaGraph Nylon Transfer Membrane 0.22 Micron, OSMONICS, USA). The first probe (MRUMtb) was specific for *M. tuberculosis* complex. The next four probes were designed to detect resistance to isoniazid:- two probes (katGwt and inhAwt) were homologous with the wild-type regions of each gene and two (katGS315T and inhAmut) were homologous with the most frequently seen mutations (the S315T mutation in the *kat*G gene and the *inh*A^{C-15T} mutation at the 5' end of a presumed ribosome binding site in the promoter of *inh*A). The next six probes (P3, P6, P9, P12, P17, and 1371A) were used to detect mutations associated with resistance to rifampicin and constitute the entire 81 bp hypervariable region (RRDR) of the *rpo*B gene.

The IP1 primer (de Beenhouwer *et al.,* (1995)) was used as a development colour control and Deskjet 690C ink (Hewlett-Packard, UK) was used for the orientation spot.

### Hybridization and colour detection:

10µl of PCR products were denatured in 10µl of Denaturation solution (400mM NaOH, 20mM EDTA). The hybridization of PCR-products to nylon strips, containing the immobilized probes, was carried out by incubation of the membranes in 500µl of hybridization buffer (5xSSPE, 0.5%SDS) at 60°C for 15 min. The strips were washed in stringent wash buffer (0.4%SSPE, 0.5% SDS) at 60°C for 10 min. The buffer was discarded and 25ml of Rinse buffer (0.1 M Tris 0.1 M NaCl, pH 7.5) were added and agitated for 1 minute in an orbital shaker. Then the buffer was discarded and this step was repeated once.

The strips were incubated for 15 min at room temperature in 5ml of SAP buffer (Rinse buffer, 0.5% B-M blocking reagent) with 25µl of alkaline phosphatase conjugated streptavidin (400µg/ml) to detect the hybridized biotinylated PCR-products. The strips were twice washed in Rinse Buffer and equilibrated in buffer 0.1M Tris, 0.1M NaCl (pH 9.5). Finally, the buffer was discarded and the membranes were incubated in 5ml of the same buffer containing 15µl of BCIP (5-bromo-4-chloro-3-indolyl phosphate) and 10µl of NBT (nitro blue tetrazolium) for 5 minutes. These chromogens served as a substrate for alkaline phosphatase producing a pattern on the strip, which could be interpreted.

### Results

### Retrospective study

A total of 465 INH-resistant isolates were analysed by the MDR screen array. Mutations in the regulatory region of the *mab*A*-inh*A were identified in 250 isolates (53.8%). Among these isolates the probe inhmut (specifically designed to detect the *inh*A^{C-15T} mutation) was positive in 240/250 isolates (96.0%). The katGS315T probe (specifically designed to detect the S315T mutation in the *kat*G gene) showed a positive hybridization signal in 161/465 (34.6%) isolates. Both the katGwt and katS315T probes failed to hybridize in 9 (1.9%) isolates. Four (0.9%) isolates showed a positive katGS315T and a negative inhAmut and inhAwt pattern. Forty-one (8.8%) isolates gave a drug susceptible profile.

### Prospective study

The MDR screen was applied to 609 clinical isolates and the results were compared with routine identification and isoniazid and rifampicin susceptibility testing.

### • Detection of M. tuberculosis complex:

From the 609 cultures received for identification and drug susceptibility analysis during the study period, PCR products were obtained from 497 cultures (81.6%). Of these 497 positive reactions, 356 (71.6%) were identified as *M. tuberculosis* complex, and 141 (28.4%) were identified as non-tuberculosis mycobacteria (NTM) by hybridisation with the macroarray. Of the 356 cultures identified genotypically as *M. tuberculosis* complex, 353 (99.2%) were confirmed as *M. tuberculosis* complex by phenotypic examination. Of the 141 genotypically defined NTM, 137 (97.2%) were confirmed phenotypically.

### • Detection of susceptible M. tuberculosis isolates

Of the 356 isolates identified as *M. tuberculosis* complex, 289 (81.2%) were identified as *M. tuberculosis* isolates susceptible to INH and RIF using the macroarray. Two hundred and seventy-seven (95.8%) were concordant with identification and routine susceptibility testing; 3 (1.1 %) were phenotypically classified as resistant to INH, and one (0.3%) was phenotypically classified as resistant to RIF.

### • Detection of M. tuberculosis rpoB, katG and mabA-inhA mutants

Of the 356 MTB complex isolates identified, 38 (10.7%) were identified as mono INH-resistant strains, 16 (4.5%) as multi-drug resistant (MTB) strains and 13 (3.6%) as mono RIF-resistant strains. Of the 38 *M. tuberculosis* isolates resistant to INH by macroarray analysis, 30 (78.9%) were correctly classified according to routine susceptibility testing, 2 (5.3%) were phenotypically classified as MDR-TB, and 5 (13.2%) were phenotypically classified as susceptible *M. tuberculosis* isolates. Of these 5 isolates, two gave negative hybridization signals for inhAwt and inhAmut probes, one isolate had a positive hybridization signal for katGwt and katGS315T probe, and 2 isolates had a positive signal for the inhAmut probe. The inhAmut probe and the katGS315T were negative for 18 and 15 isolates respectively. Five isolates gave a negative reaction with both probes.

Among the 13 *M. tuberculosis* isolates resistant to RIF alone, 8 (61.5%) were phenotypically identified as resistant to RIF, 4 (30.8%) were phenotypically identified as MDR-TB and 1 (7.7%) was phenotypically identified as susceptible (a negative hybridization signal for the P3 and P6 probes).

All 16 MDR-TB were phenotypically identified as MDR-TB. Of these 16 isolates, 12 had a positive signal for katGS315T, 1 for inhAmut and 3 for both probes.

Considering all strains defined as either INH or RIF resistant by macroarray, 48/54 (88.9%) were concordant for INH and 28/29 (96.6%) for RIF resistance.

### Discussion

The MDR screen macroarray described herein presents a rapid and sensitive method for detecting *M. tuberculosis* and to determine INH and RIF susceptibility in clinical isolates. The basic principle of the MDR screen array developed in this study is that each nucleotide change should block the hybridization of the target with the corresponding wild-type probes (P3, P6, P9, P17, 1371A, *kat*Gwt and *inh*Awt probes), or permit the hybridization of the target and the corresponding mutant probe (*kat*GS315T and *inh*Amut probes).

The PCR reaction was positive for detection of *M. tuberculosis* complex in 356 out of 363 isolates (sensitivity 98.1 %) from patients who were later diagnosed by conventional techniques. Eight isolates, classified as *M. tuberculosis* by routine identification procedures, were not identified by the macroarray. Only 1 isolate out of 356 positive PCR for the *M. tuberculosis* complex was phenotypically identified as NTM (specificity 99.7%).

The MDR screen macroarray results were concordant with conventional identification and susceptibility testing results for 331 out of 356 *M. tuberculosis* cases (93.0%) and 137 out of 141 NTM isolates (97.2%). Some of the discrepant isolates displayed wild type array patterns but were phenotypically classified as resistant (3 resistant to isoniazid and one resistant to rifampicin), or they were phenotypically classified as MDR-TB and had a mono-RIF or mono-INH resistant macroarray pattern. Previous studies have demonstrated that approximately 4% of RIF-resistant and 30-40% of INH-resistant isolates have no mutations within the 81-bp region of the *rpo*B gene (the region associated with RIF resistance) and within the regulatory region of the *mab*A*-inh*A operon/ within the *kat*G gene, respectively.

More than 95% of RIF-resistant strains are associated with mutations within an 81-bp region of the *rpo*B gene. The array used in this study is able to detect known mutations, including point mutations, insertions and deletions, because the probes tiled on the array constitute the entire 81 bp wild type hypervariable region. This macroarray has the potential to be used widely as there are no significant differences in the distribution of *rpo*B mutations globally.

The array used in this study is simple to perform and interpret, requiring only a basic knowledge of molecular biology to perform it successfully. Although DNA sequencing is simple for laboratories already performing it routinely, the costs of equipment and maintenance do not make it a cost-effective option for many clinical laboratories. The cost of our array is an important factor for its widespread applicability. This array advantageously costs less than $5, whereas the commercial INNOLiPA kit costs $720 and only detects resistance to RIF.

The potential of the MDR macroarray for testing different targets has been demonstrated in this study. The array described here can be expanded to detect other specific mutations in the *kat*G gene. Epidemiological markers could also be added to the array for tracing epidemic or sporadic dissemination of strains.

### Example 4

### Bacterial isolates

A panel of 40 *M. tuberculosis* isolates was assembled in order to give a range of genotypes genotype at the *rpoB* RRDR, *katG*315 and *mabA-inh*⁻¹⁵ loci. These isolates were cultured on LJ and drug susceptibility testing was performed using the resistance ratio method on Lowenstein-Jensen media.

### Preparation of DNA extracts

Cell paste from LJ medium was suspended in 100µl purified water and an equal volume of chloroform was added. The tubes were heated at 80°C for 20 minutes, placed in the freezer for 5 minutes and mixed briefly using a vortex mixer. Immediately before use as PCR template tubes were centrifuged for 3 minutes at 12000xg.

### PCR

Biotinylated target PCR products were generated in a 20 µl multiplex PCR. This contained 1 x Ammonium reaction buffer (Bioline Ltd., London UK), dNTP at 0.2mM each (Amersham Biosciences, Chalfont St Giles, UK), MgCl₂ at 1.5mM (Bioline), primers KatGP5IO and KatGP6BIO at 0.25mM, primers INHAP3BIO, TOMIP2BIO, FTP1BIO and BrpoB1420R at 1mM (ThermoHybaid, Ulm, Germany), 1 unit Taq-polymerase (Bioline) and 1 µl of DNA template. Primer sequences are given in table 2. Thermal cycling was performed on a Perkin Elmer 9700 Thermocycler using the following program: 5mins at 95°C, 30X(30secs at 65°C, 60secs at 72°C), hold 5mins at 72°C.

### Construction of MDR screen macroarray

The 10 probes illustrated in Table 1, above, were used to produce a macroarray, together with an additional probe that targeted an *M. tuberculosis* complex specific locus of *M. tuberculosis rpoB.* Probes 1-4 of Table 1 are designed to analyse loci associated with INH resistance. Probes 1 and 3 (K315WTC and tomiwt), detect the wild-type (WT) genotypes at *katG*315 and at *mabA-inhA*⁻¹⁵, whilst Probes 2 and 4 (K315GC and tomimut1), detect the most frequently seen genotype at each locus, *katG*315^{AGC→ACC} and *mabA-inhA* ^{-15C→T} respectively. Probes 6-10 (MRURP3, MRURP6, MRURP9, MRURP12, MRURP17 and MRU1371A) formed a scanning array for detection of the WT genotype of the RRDR of *M. tuberculosis rpoB.* In order to optimise probe performance within the array oligonucleotide probes were synthesised with 3' poly-T tails. Oligonucleotide probes (Invitrogen, Paisley UK) were diluted to 20µM in water containing 0.001 % bromophenol blue and applied to nylon membrane (Magnagraph 0.22µM, Osmonics, Minnetonka USA) using a handheld arraying device (VP Scientific, San Diego USA).

In addition to the probes, a permanent ink spot was applied to the membrane in order to orientate the array and a spot of primer FTIP1BIO at 2µM as a colour development control. Probes were UV-crosslinked to the nylon membrane. The membranes were washed in 0.5% 20XSSC (Sigma, Poole, UK) then dried, cut and placed in 2 ml polythene hybridization tube (Alpha Labs, Eastleigh, UK).

### Hybridisation and colour detection

The biotin labelled PCR products were denatured by adding an equal volume of denaturation solution (0.4M NaOH, 0.02M EDTA) and incubating at room temperature for 15 minutes. A 20µl aliquot of the denatured PCR was added to tube containing an array and 500µl hybridization solution (5xSSPE; 0.5% SDS), which was agitated in a hybridization oven at 60°C for 15 minutes. The strips were then washed in wash buffer (0.4%SSPE, 0.5% SDS) at 60°C for 10 min in the hybridization oven. The arrays were now agitated in rinse buffer (0.1 M Tris 0.1 M NaCl, pH 7.5) at room temperature (RT) for 1 minute. This rinse step was repeated then once more using the rinse buffer containing 0.1 % blocking reagent (Roche, Lewes UK). The arrays were now agitated at RT for 15 minutes in the rinse buffer with 0.1 % blocking reagent and 1/25 dilution of streptavidin-alkaline phosphatase conjugate at 400µg/ml (BioGenex, San Ramon USA). The membranes were then washed twice in wash solution and once in substrate buffer (0.1 M Tris, 0.1 M NaCl at pH 9.5) before being incubated at RT for 5 minutes in substrate buffer containing 0.34mg/ml NBT (USB, Cleveland, USA) and 0.17mg/ml BCIP (USB). The membranes were washed in water before being air-dried and the hybridization patterns noted.

### Interpretation of the macroarray

Hybridization to any of the probes directed towards *rpoB* is indicative of a WT genotype at that locus, conversely lack of hybridization with a given *rpoB* probe is indicative of a mutant genotype at that locus. Hybridization with K315WTC is indicative of a *katG*315 WT genotype whereas absence is indicative of a mutant genotype at this or surrounding this locus. Absence of hybridization with K315WTC and hybridization with K315GC is indicative of the katG315 AGC>ACC genotype. Likewise, hybridization with TOMIWT is indicative of a *mabA-inhA*⁻¹⁵ WT genotype whereas absence is indicative of a mutant genotype at this or surrounding this locus. Absence of hybridization with TOMIWT and hybridization with TOMIMUT1 is indicative of the *mabA-inhA*^{-15C→T} genotype.

### DNA sequencing

Single primer pairs (see Table 2, above) were used to generate single *rpoB, katG* or *inhA* PCR products using the method given above. These were diluted 1/100 in purified water and sequenced using CEQ Quick Start sequencing kits and a CEQ 8000 instrument (Beckman Coulter, High Wycombe, UK) according to the manufacturers instructions. The PCR products were sequenced in both directions using the amplification primers given in Table 2, above.

### Results

The panel of 40 *M. tuberculosis* isolates contained 30 MDR isolates, 5 RIF-mono-resistant isolates, 1 INH mono-resistant isolate and 4 isolates sensitive to RIF and INH. Sequencing of the RRDR of *rpoB* of these isolates revealed 36 different genotypes in addition to the WT. Analysis of the codons most commonly associated with RIF resistance showed two different mutations at the codon 531, 6 different mutations at the codon 526, and 4 different mutations at the codon 516. Mutations in codons 509, 511, 513, 515, 522, 528, 529 and 533 were also seen. Seven isolates contained two separate single base substitutions, four isolates contained insertions and three contained deletions. The *katG*315 and *mabA-inh*A⁻¹⁵ genotype of 28 of the isolates were determined. Three genotypes in addition to the WT were seen at *katG*315 and a C to T substitution at *mabA-inh*A⁻¹⁵ was seen in addition to the WT. The genotypes of individual isolates are shown in Table 5, below.

**Table 5**

| Isolate | Susceptibility by phenotype | | *katG315*/ *mabA-inhA⁻¹⁵* genotype | *rpoB* genotype | Non-hybridizing probes | Susceptibility by array | |
|---|---|---|---|---|---|---|---|
| | INH | RIF | | | | INH | RIF |
| 01/07786 | R | R | | 1302C>G / S509R + 1351C>T / H526Y | P2 P5 P10 | R | R |
| 236-02 | R | R | AGC>ACC /WT | 1307T>C / L511P + 1322A>G / D516G | P2 P5 P8 P7 | R | R |
| 98/05219 | S | R | | 1307T>C / L51 1 P + 1351C>G / H526D | P3 P5 P6 P10 | S | R |
| 2936-99 | R | R | WT/WT | 1312C>A / Q513K | P3 P5 P6 P7 | S | R |
| Is20043 | R | R | | 1313A>C / Q513P | P2 P5 P6 P7 | R | R |
| 98/05844 | R | R | AGC>AAC/ WT | 1313A>T / Q513L | P2 P3 P5 P6 P7 | R | R |
| 02/07435 | R | R | | 1314 CCAACT ins 513 | P2 P5 P6 P7 | R | R |
| 2651-96 | R | R | AGC>ACC /WT | 1315 TTC ins 514 | P2 P5 P6 P7 | R | R |
| Is14373 | R | R | | 1315-1323 Del TTCATGGAC 514-516 | P2 P5 P6 P7 | R | R |
| Is11195 | R | R | WT/WT | 1316-1318 Del TCA 514-515 | P3 P5 P6 P7 | S | R |
| Is14786 | R | R | | 1318A>G / M515V + 1351C>A / H526N | P3 P4 P6 P7 P10 | R | R |
| 1763-97 | R | R | AGC>AAC/ WT | 1318lns ATTCAT 515 | P2 P3 P5 P6 P7 | R | R |
| 98/07530 | R | R | | 1320G>A / M515I | P2 P5 P7 P8 | R | R |
| 2883-97 | R | R | AGC>ACC /WT | 1321-26 Del GACCAG 516-517 | P2 P5 P6 P7 P8 | R | R |
| Is11125 | R | R | AGC>ACC /WT | 1321-2GA>TT / D516F | P2 P4 P7 | R | R |
| 1579-96 | S | R | WT/WT | 1321G>T / D516Y | P3 P5 P7 | S | R |
| Is14027 | R | R | | 1322A>G / D516G | P3 P4 P7 P10 | R | R |
| 1004-01 | R | R | AGC>ACA/ WT | 1322A>T / D516V | P5 P7 | R | R |
| 1071-98 | R | R | AGC>ACC /WT | 1322A>T / D516V + 1351C>G / H526D | P2 P5 P6 P7 P8 P10 | R | R |
| 98/00699 | R | R | | 1334 AGAACAACC ins 520 | P3 P4 | R | S |
| 1992-00 | R | R | WT/WT | 1339-40TC>CA / S522Q | P3 P5 P9 | S | R |
| 1445-01 | R | R | AGC>ACC /WT | 1340C>G / S522W | P2 P5 P9 | R | R |
| 395-98 | R | R | AGC>ACC /WT | 1340C>T / S522L | P2 P5 P9 | R | R |
| 03/02007 | R | R | WT/WT | 1350-1 CC>TT / T525 + 1351C>T / H526Y | P3 P5 P10 | S | R |
| 2323-02 | R | R | AGC>ACC /WT | 1351-2CA>TG / H526C | P2 P5 P10 | R | R |
| 1828-00 | R | R | WT/inhA C-15T | 1351C>G / H526D | P3 P4 P10 | R | R |
| 2031-02 | S | R | WT/WT | 1351C>T / H526Y | P3 P5 P10 | S | R |
| 3381-97 | R | R | AGC>ACC /WT | 1352 A>G / H526R | P2 P5 P10 | R | R |
| 740-97 | R | R | AGC>ACC /WT | 1352A>C / H526P | P2 P5 P10 | R | R |
| 1810-96 | R | R | AGC>AAC/ WT | 1352A>T / H526L | P2 P3 P5 P10 | R | R |
| 01/03682 | S | S | | 1359C>T / R528R | P3 P5 P10 | S | R |
| 02/06539 | S | R | | 1361G>C / R529P | P3 P5 P10 | S | R |
| 1255-98 | R | R | WT/inhA C-15T | 1363C>A / L530M + 1367C>T / S531 L | P3 P4 P6 P7 P11 | R | R |
| 01/11196 | R | R | | 1367C>G / S531W | P2 P5 P11 | R | R |
| Is5 | R | R | WT/WT | 1367C>T / S531L | P3 P5 P11 | S | R |
| 02/03056 | S | R | WT/WT | 1373T>C / L533P | P3 P5 P11 | S | R |
| 03/06044 | S | S | WT/WT | WT | P3 P5 | S | S |
| 03/04307 | S | S | WT/WT | WT | P3 P5 | S | S |
| 03/05269 | S | S | WT/WT | WT | P3 P5 | S | S |
| 1182-01 | R | S | AGC>ACA/ WT | WT | P5 | R | S |

The crude DNA extracts from each of the isolates in the panel were analysed using the MDR screen macroarray, the design of which is shown in Figure 4, as are representative examples of the developed arrays. All isolates produced interpretable hybridization patterns with the array and 39 from the 40 detected mutations when they were present. The one isolate that which failed to give a mutant genotype using the array contained a nine base insertion in the *rpoB* RRDR. All other isolates were correctly identified as mutant or wild type. A mutation was detected in all 35 of the RIF resistant isolates. A mutation was also detected in a RIF susceptible isolate that did indeed carry a synonymous mutation. The array detected mutations at katG315 or *mabA-inhA⁻¹⁵* in twenty-seven out of the 31 INH resistant isolates, the remaining four were wild type at these loci.

Amino acid codons 516, 526 and 531 are the most prevalent codons involved in rifampin resistance. These three codons may be responsible for 80% of RIF-resistant M. *tuberculosis* cases. All the isolates with mutations in these positions were correctly identified. The rpoB531, rpoB526 and rpo516 mutant alleles showed a negative hybridization signal for their respective probes (see Figure 4B, patterns 4, 1 and 7).

Others less frequent mutations at the 511, 513, 515, 522, 529 and 533 codons and 6 double single mutation, 3 different deletions and two insertions were correctly identified. Only one strain with an insertion of 9 nucleotides (AGAACAACC) at the codon 520 was incorrectly identified.

Four MDR-resistant isolates showed a wild type pattern for isoniazid resistance with positive hybridization signal for the katGwt and inhA probes: This is possible from the resistance to isoniazid is caused by a variety of mutations at several chromosome loci of M. *tuberculosis.* Mutations in the *katG* and the regulatory region of the *mabA-inhA* operon have not been found in approximately 30-50% of the INH-resistant M. *tuberculosis* isolates.

All the isolates with known sequences of the part relevant of the *katG* gene and the regulatory region of the *mabA-inhA* operon were correctly identified. The INH-resistant M. *tuberculosis* isolates with different mutations in the 315 amino acid position in the *katG* gene were correctly identified. The INH-resistant isolates with the S315T mutation had a pattern with a negative hybridization signal for the katGwt and a positive hybridization signal for the katGmut probe (Figure 1 B(2)). Others different mutations (S315 ACA, S315 AAC or S315 AGG) showed a pattern with a negative hybridization signal for both probes (Figure 1B(5)). The INH-resistant M. *tuberculosis* isolates with the *inhA^{C-15T}* mutation showed a negative hybridization signal for the inhAwt probe and a positive hybridization signal for the inhAmut probe (Figure 1 B(6)).

### Discussion

Detecting drug resistance in M. *tuberculosis* isolates by determining genotype is an attractive alternative to conventional phenotypic susceptibility testing because results can be generated within hours with minimal manipulation of live organism. Obviously this approach can only be used where genotypic markers for drug resistance have been identified. This is the case for MDRTB where a range of mutations in the RRDR of *rpoB* are highly specific to RIF resistant isolates and 2 point mutations, one in *katG* and one associated with *inhA* are highly specific to INH resistant isolates. By analysing these 3 different loci MDRTB can be identified. Using macroarray analysis all these loci can be analysed in parallel. We have described such a macroarray-based assay for the detection of MDRTB above. The principle of the MDR screen array assay is that a mutation should impede the hybridization of the target to the relevant WT probe or in the case of the katG315 or inhA loci permit the hybridisation to the corresponding mutant probe. This was capable of detecting 35/36 different mutations in the RRDR of *rpoB,* 3/3 different mutations at katG315 and 1/1 at inhA⁻¹⁵.

When susceptibility is designated by genotype, there are three sources of discrepancy with the more definitive phenotypic testing. Firstly, a resistant isolate may not contain the marker. According to the literature, this is seen in <5% of RIF resistant isolates and between 10 and 30% of INH resistant isolates using the marker used in this study. This type of discrepancy was seen in 4 INH resistant isolates in the present study. Identifying further markers and including these in the assay would reduce these discrepancies. Secondly, a susceptible isolate may contain synonymous mutation which when detected would lead to the isolate being designated resistant. Any discrepancies caused by synonymous mutations at the loci used in this assay may be reduced by identification of said mutations either by sequencing the mutant loci or by inclusion on the macroarray of probes directed at all possible mutations. One *rpoB* mutant such as this was seen in the present study. A third source of discrepancy is failure to correctly detect mutations present. This type of discrepancy is minimised in this array by careful selection of the probes used. Because the hybridisation behaviour of a given probe and target combination is difficult to predict, it is essential to validate all probes with potential targets. In the present study only one mutation was not detected. This was a 9 base insertion which had the effect of producing a 3 base mismatch at the 5' end of the 22 base probe MRURP9 (Probe 7 of Table 1) which presumably did not destabilise the hybridisation duplex sufficiently to prevent the detection of hybridization.

In summary, the above-described MDR-screen macroarray identified M. *tuberculosis* complex isolates resistant to isoniazid and/or rifampicin, the two most important drugs in the treatment of tuberculosis. The assay is easy to perform and interpret and could be implemented into the routine practices of clinical laboratories although most usefully in areas with a high prevalence of MDR M. *tuberculosis.*

The above Examples demonstrate the broad applicability of macroarrays comprising probes according to the present invention for the detection of mutations consistent with RIF and INH resistance in *Mycobacterium* species, in particular, in members of the *Mycobacterium tuberculosis* complex, such as M. *tuberculosis.* This system is simple and safe to use, and enables rapid identification of MDRTB. The present assay therefore leads to the earlier institution of appropriate chemotherapy, thereby improving the probability of individual cure and generally improving public health.

### References

Kruuner A, Hoffner SE, Sillastu H, Danilovits M, Levina K, Svenson SB et.al. Spread of drug-resistant pulmonary tuberculosis in Estonia. J.Clin. Microbiol. 2001; 39: 3339-45
World Health Organization, International Union Against Tuberculosis. Antituberculosis drug resistance in the world. Geneva, Switzerland, 2000.
Pablos-Mendez A, Raviglione MC, Laszlo A, et al. Global surveillance for antituberculosis-drug resistance, 1994-1997. N Engl J Med 1998; 338:1641-9
Perelman MI. TB control analysis in Russia in 2001. Problemy tuberculeza. 2003; 2:3-11 (in Russian).
KruunerA, Sillastu H, Danilovits M, Levina K, Svenson SB Hoffner SE, Kallenius G. et.al. Drug resistant tuberculosis in Estonia. Int. J.Tuberc. Lung. Dis. 1998; 2: 130-33
Toungoussova OS, Sandven P, Mariandyshev AO, Nizovtseva NI, Bjune G, Caugant DA. Spread of drug-resistant Mycobacterium tuberculosis strains of the Beijing genotype in the Archangel Oblast, Russia. J Clin Microbiol. 2002;40:1930-7
Drobnievski FA, Balabanova YM, Ruddy M, Weldon L, Jeltkova K, Brown T. et al. Rifampin- and Multidrug-resistant tuberculosis in Russian civilians and prison inmates: dominance of the Beijing strain family. Emerg. Infect. Dis. 2002; 8:1320-1326
Mokrousov I, Narvskaya O, Otten T, et al. High prevalence of KatG Ser315Thr Substitution among Isoniazid-Resistant Mycobacterium tuberculosis Clinical Isolates from Northwestern Russia, 1996 to 2001. Antimicr Agent Chemother 2002; 46: 1417-24
Munsiff SS, Bassoff T, Nivin B, Li J, Sharma A, Bifani P, Mathema B et al. Molecular epidemiology of multidrug-resistant tuberculosis, New York City, 1995-1997. Emerg Infect Dis. 2002; 8:1230-8
Agerton T,Valway SE, Blinkhorn RJ, Shilkret KL, Reves R, Schluter WW et al. Spread of strain W, a highly drug-resistant strain of Mycobacterium tuberculosis, across the United States. Clin. Infect. Dis. 1999; 29: 85-92
Breathnach AS, De Ruiter A. Holdsworth GMC, Bateman NT, O'Sulliovan DGM Rees PJ, et al. An outbreak of multi-drug resistant tuberculosis in a London teaching hospital. J.Hosp. Infect. 1998; 39:111-7
Espinal MA. The global situation of MDR-TB. Tuberculosis 2003; 83:44-51
TB microbiological diagnostics standardization. USSR Ministry of healthcare Order No 558, 8/06/1978. (in Russian).
Rattan A, Kalia A, Ahmad N. Multidrug-resistant Mycobacterium tuberculosis: molecular perspectives. Emerg Infect Dis. 1998;4:195-209
Bodmer T, Zurcher K, Imboden P, Telenti A. Mutation position and type of substitution in the b-subunit of the RNA polymerase influence in-vitro activity of rifampicins in Rifampicin-resistant Mycobacterium tuberculosis. J. Antimicr. Chemother. 1995; 35:345-48
Fluit AC, Visser MR, Schmitz RJ. Molecular Detection of Antimicrobial Resistance. Clin. Microbiol.Rev. 2001; 14: 836-71
Musser JM Antimicrobial agent resistance in Mycobacteria: molecular genetic insights. Clin. Micr. Rev.1995; 8:496-514
Alvarado-Esquivel C, Rossau R, Martinez-Garcia S et al. Characterization of rpoB gene mutations in rifampicin resistant Mycobacterium tuberculosis strains isolated from pulmonary tuberculosis patients at 5 Mexican public hospitals. Rev Invest Clin 2001; 56:526-530
Bártfal Z, Somoskövi A, Ködmön C et al. Molecular Characterization of rifampin-resistant isolates of Mycobacterium tuberculosis from Hungary by DNA sequencing and the line probe assay. Clin Microbiol. 2001; 39:3736-39
Zhang Y., Heym B., Allen B. et al. The Catalase-peroxydase Gene and isoniazid Resistance of Mycobacterium tuberculosis. Nature. 1992; 358: 591-93
Wilson TM, Collins DM. AhpC, a gene involved in izoniazid resistance of the Mycobacterium tuberculosis complex. Molecular Microbiology. 1996; 19: 1025-34
Garcia de Viedma G. Rapid detection of resistance in Mycobacterium tuberculosis: a review discussing molecular approaches. Clin Microbiol Infect. 2002; 9:349-359
van Rie A, Warren R, Mshanga I et al. Analysis for a limited number of gene codons can predict drug resistance of Mycobacterium tuberculosis in a high-incidence community. Clin. Microbiol. 2001; 39: 636-641.
Collins CH, Grange JM, Yates MD. Tuberculosis bacteriology. Organization and Practice. 2nd Edition. 1997; Oxford: Butterworth-Heinemann.
Yates MD, Drobniewski FA, Wilson SM Evaluation of a rapid PCR-based epidemiological typing method for routine studies of Mycobacterium tuberculosis J.Clin. Microbiol 2002; 40:712-4.
Ahmad, S., E. Fares, G. F. Araj, T. D. Chugh, and A. S. Mustafa. 2002. Prevalence of S315T mutation within the katG gene in isoniazid-resistant clinical Mycobacterium tuberculosis isolates from Dubai and Beirut. Int. J. Tuberc. Lung Dis. 6: 920-926.
Bakonyte, D., A. Baranauskaite, J. Cicenaite, A. Sosnovskaya, and P. Satakenas. 2003. Molecular characterization of isoniazid-resistant Mycobacterium tuberculosis clinical isolates in Lithuania. Antimicrob. Agents Chemother. 47: 2009-20011
Martilla, H., H. Soini, E. Eerola, E. Vyshnevskaya, B. Vyshnevskiy, T. Otten, A. Vasilyef, and M. Viljanen. 1998. A Ser315Thr substitution in KatG is predominant in genetically heterogeneous multidrug-resistant Mycobacterium tuberculosis isolates originating from the St. Petersburg area in Russia. Antimicrob. Agents Chemother. 42: 2443-2445.
Tracevska, T., I. Jansone, L. Broka, O. Marga, and V. Baumanis. 2002. Mutations in the rpoB and katG genes leading to drug resistance in Mycobacterium tuberculosis in Latvia. J. Clin. Microbiol. 40: 3789-3792.
Narvskaya, O., Otten, T., Limeschenco, E., Sapozhnikova, N., Graschenkova, N., Steklova, L., Nikonova, A., Filipenko, M.L., Mokrousov, I., Vyshnevskiy. B., 2002. Nosocomial outbreak of multidrug-resistant tuberculosis caused by a strain of Mycobacterium tuberculosis W-Beijing family in St. Petersburg Russia. Eur. J. Clin Microbiol. Infect Dis. 21, 596-602.
Davies, P.D., 2003. The worldwide increase in tuberculosis: how demographic changes, HIV, infection and increasing numbers in poverty are increasing tuberculosis. Ann. Med. 35-235-243.
De Beenhouwer, H., Liang, Z., Jannes, G., Mijs, W., Machtelinckx, L., Rossau, R., Traore, H., Portaels, F., 1995. Rapid detection of rifampin resistance in sputum and biopsy specimens from tuberculosis patients by PCR and line probe assay. Tubercle Lung. Dis. 76, 425-430.
Collins CH, Grange JM, Yates MD. Tuberculosis bacteriology. Organization and Practice. 2nd Edition. 1997; Oxford: Butterworth-Heinemann.
Brown, T., Anthony,R., 2000.The addition of low numbers of 3' thymine bases can be used to improve the hybridization signal of oligonucleotides for use within arrays on nylon supports. J Micro Methods. 42,203-207.
Bifani, P.J., Plikaytis, B., Kapur, V., Stockbauer, K., Pan, X., Lutfey, M.L., Moghazeh, S.L., Eisner, W., Daniel, T.M, Kaplan, M.H., Crawford, J.T., Musser, J.M., Kreiswirth, B.N., 1996. Origin and interstate spread of New York City multidrug-resistant Mycobacterium tuberculosis clone family. JAMA. 275,452-457.

## Claims

1. A set of nucleic acid probes for use in an assay for detecting multi-drug resistant *Mycobacterium sp.* in a sample, which set includes:
probe 1 comprising a nucleic acid sequence of 10 nucleotides that binds to a first target sequence ACCAGCGGCA, or to the complement thereof;
probe 2 comprising a nucleic acid sequence of 10 nucleotides that binds to a second target sequence GCCGGTGGTG, or to the complement thereof;
probe 3 comprising a nucleic acid sequence of 10 nucleotides that binds to a third target sequence TATCGTCTCG, or to the complement thereof;
probe 4 comprising a nucleic acid sequence of 10 nucleotides that binds to a fourth target sequence TATCATCTCG, or to the complement thereof;
probe 5 comprising a nucleic acid sequence of 10 nucleotides that binds to a fifth target sequence GAATTGGCTC, or to the complement thereof;
probe 6 comprising a nucleic acid sequence of 10 nucleotides that binds to a sixth target sequence CTGGTCCATG, or to the complement thereof;
probe 7 comprising a nucleic acid sequence of 10 nucleotides that binds to a seventh target sequence GGTTGTTCTG, or to the complement thereof;
probe 8 comprising a nucleic acid sequence of 10 nucleotides that binds to an eighth target sequence CCCGACAGCG, or to the complement thereof;
probe 9 comprising a nucleic acid sequence of 10 nucleotides that binds to a ninth target sequence GCTTGTGGGT, or to the complement thereof; and
probe 10 comprising a nucleic acid sequence of 10 nucleotides that binds to a tenth target sequence CCAGTGCCGA, or to the complement thereof;
wherein each of said probes is 18-25 nucleotides long;
and wherein, in use, once a probe is bound to a respective target sequence, a detectable signal is provided.

2. A set of probes according to Claim 1, which set includes:-
probe 1 comprising a nucleic acid sequence of 15 nucleotides that binds to a first target sequence ATCACCAGCGGCATC, or to the complement thereof;
probe 2 comprising a nucleic acid sequence of 15 nucleotides that binds to a second target sequence GATGCCGGTGGTGTA, or to the complement thereof;
probe 3 comprising a nucleic acid sequence of 15 nucleotides that binds to a third target sequence ACCTATCGTCTCGCC, or to the complement thereof;
probe 4 comprising a nucleic acid sequence of 15 nucleotides that binds to a fourth target sequence ACCTATCATCTCGCC, or to the complement thereof;
probe 5 comprising a nucleic acid sequence of 15 nucleotides that binds to a fifth target sequence ATGAATTGGCTCAGC, or to the complement thereof;
probe 6 comprising a nucleic acid sequence of 15 nucleotides that binds to a sixth target sequence GTTCTGGTCCATGAA, or to the complement thereof;
probe 7 comprising a nucleic acid sequence of 15 nucleotides that binds to a seventh target sequence GCGGGTTGTTCTGGT, or to the complement thereof;
probe 8 comprising a nucleic acid sequence of 15 nucleotides that binds to an eighth target sequence AACCCCGACAGCGGG, or to the complement thereof;
probe 9 comprising a nucleic acid sequence of 15 nucleotides that binds to a ninth target sequence GGCGCTTGTGGGTCA, or to the complement thereof;
probe 10 comprising a nucleic acid sequence of 15 nucleotides that binds to a tenth target sequence GCCCCAGTGCCGACA, or to the complement thereof.

3. A set of probes according to Claim 1 or Claim 2, which set includes:- probe 1 comprising the sequence TGCCGCTGGT, or a sequence having at least 90% sequence identity thereto;
probe 2 comprising the sequence CACCACCGGC, or a sequence having at least 90% sequence identity thereto;
probe 3 comprising the sequence CGAGACGATA, or a sequence having at least 90% sequence identity thereto;
probe 4 comprising the sequence CGAGATGATA, or a sequence having at least 90% sequence identity thereto;
probe 5 comprising the sequence GAGCCAATTC, or a sequence having at least 90% sequence identity thereto;
probe 6 comprising the sequence CATGGACCAG, or a sequence having at least 90% sequence identity thereto;
probe 7 comprising the sequence CAGAACAACC, or a sequence having at least 90% sequence identity thereto;
probe 8 comprising the sequence CGCTGTCGGG, or a sequence having at least 90% sequence identity thereto;
probe 9 comprising the sequence ACCCACAAGC, or a sequence having at least 90% sequence identity thereto;
probe 10 comprising the sequence TCGGCACTGG, or a sequence
having at least 90% sequence identity thereto;
wherein the underlined nucleotides within the sequences of probes 1, 2, 3 and 4 are essential, and may not be substituted by any other nucleotide.

4. A set of probes according to Claim 3, wherein:-
probe 1 comprises the sequence GATGCCGCTGGTGAT, or a sequence having at least 90% sequence identity thereto;
probe 2 comprises the sequence ATCACCACCGGCATC, or a sequence having at least 90% sequence identity thereto;
probe 3 comprises the sequence GGCGAGACGATAGGT, or a sequence having at least 90% sequence identity thereto;
probe 4 comprises the sequence GGCGAGATGATAGGT, or a sequence having at least 90% sequence identity thereto;
probe 5 comprises the sequence AGCTGAGCCAATTCATG, or a sequence having at least 90% sequence identity thereto;
probe 6 comprises the sequence AATTCATGGACCAGAACA, or a sequence having at least 90% sequence identity thereto;
probe 7 comprises the sequence ACCAGAACAACCCGC, or a sequence having at least 90% sequence identity thereto;
probe 8 comprises the sequence ACCCGCTGTCGGGGTT, or a sequence having at least 90% sequence identity thereto;
probe 9 comprises the sequence TGACCCACAAGCGCC, or a sequence having at least 90% sequence identity thereto;
probe 10 comprises the sequence CTGTCGGCACTGGGGCC, or a
sequence having at least 90% sequence identity thereto;
wherein the underlined nucleotides within the sequences of probes 1, 2, 3 and 4 are essential, and may not be substituted by any other nucleotide.

5. A set of probes according to any previous Claim, which set includes:- probe 1 comprising the sequence SEQ ID NO 1, or a sequence having at least 90% sequence identity thereto;
probe 2 comprising the sequence SEQ ID NO 2, or a sequence having at least 90% sequence identity thereto;
probe 3 comprising the sequence SEQ ID NO 3, or a sequence having at least 90% sequence identity thereto;
probe 4 comprising the sequence SEQ ID NO 4, or a sequence having at least 90% sequence identity thereto;
probe 5 comprising the sequence SEQ ID NO 5, or a sequence having at least 90% sequence identity thereto;
probe 6 comprising the sequence SEQ ID NO 6, or a sequence having at least 90% sequence identity thereto;
probe 7 comprising the sequence SEQ ID NO 7, or a sequence having at least 90% sequence identity thereto;
probe 8 comprising the sequence SEQ ID NO 8, or a sequence having at least 90% sequence identity thereto;
probe 9 comprising the sequence SEQ ID NO 9, or a sequence having at least 90% sequence identity thereto;
probe 10 comprising the sequence SEQ ID NO 10, or a sequence having at least 90% sequence identity thereto;
wherein nucleotide residue 11 within SEQ ID NOs 1 and 2 is essential and may not be substituted by any other nucleotide;
and wherein nucleotide residue 8 within SEQ ID NOs 3 and 4 is essential and may not be substituted by any other nucleotide.

6. A set of nucleic acid probes for use in an assay for detecting multi-drug resistant *Mycobacterium sp.* in a sample, which set includes:-
probe 1 comprising a nucleic acid sequence of 10 nucleotides that binds to a first target sequence ACCAGCGGCA, or to the complement thereof;
probe 2 comprising a nucleic acid sequence of 10 nucleotides that binds to a second target sequence GCCGGTGGTG, or to the complement thereof;
probe 5 comprising a nucleic acid sequence of 10 nucleotides that binds to a fifth target sequence GAATTGGCTC, or to the complement thereof;
probe 6 comprising a nucleic acid sequence of 10 nucleotides that binds to a sixth target sequence CTGGTCCATG, or to the complement thereof;
probe 7 comprising a nucleic acid sequence of 10 nucleotides that binds to a seventh target sequence GGTTGTTCTG, or to the complement thereof;
probe 8 comprising a nucleic acid sequence of 10 nucleotides that binds to an eighth target sequence CCCGACAGCG, or to the complement thereof;
probe 9 comprising a nucleic acid sequence of 10 nucleotides that binds to a ninth target sequence GCTTGTGGGT, or to the complement thereof; and probe 10 comprising a nucleic acid sequence of 10 nucleotides that binds to a tenth target sequence CCAGTGCCGA, or to the
complement thereof;
wherein each of said probes is 18-25 nucleotides long;
and wherein, in use, once a probe is bound to a respective target sequence, a detectable signal is provided.

7. A set of probes according to Claim 6, which set includes:-
probe 1 comprising the sequence TGCCGCTGGT, or a sequence having at least 90% sequence identity thereto;
probe 2 comprising the sequence CACCACCGGC, or a sequence having at least 90% sequence identity thereto;
probe 5 comprising the sequence GAGCCAATTC, or a sequence having at least 90% sequence identity thereto;
probe 6 comprising the sequence CATGGACCAG, or a sequence having at least 90% sequence identity thereto;
probe 7 comprising the sequence CAGAACAACC, or a sequence having at least 90% sequence identity thereto;
probe 8 comprising the sequence CGCTGTCGGG, or a sequence having at least 90% sequence identity thereto;
probe 9 comprising the sequence ACCCACAAGC, or a sequence having at least 90% sequence identity thereto;
probe 10 comprising the sequence TCGGCACTGG, or a sequence
having at least 90% sequence identity thereto;
wherein the underlined nucleotides within the sequences of probes 1 and 2 are essential, and may not be substituted by any other nucleotide.

8. A set of probes according to any previous claim, wherein one or more of probes 5, 6, 7, 8, 9 and 10 is replaced by one or more probes having at least 90% sequence identity to a sequence selected from SEQ ID NOs: 11-32.

9. A set of probes according to any of Claims 1-7, further comprising one or more probes having at least 90% sequence identity to a sequence selected from SEQ ID NOs: 11-32

10. A set of probes according to any previous Claim wherein said probes are immobilised onto a solid support.

11. A set of probes according to any previous Claim wherein said probes each have a 3' poly-T tail.

12. A set of probes according to any previous Claim wherein said multi-drug resistant *Mycobacterium sp.* are multi-drug resistant members of the *Mycobacterium tuberculosis* complex (MTC).

13. A set of probes according to Claim 10, wherein said multi-drug resistant *Mycobacterium sp.* are multi-drug resistant *Mycobacterium tuberculosis.*

14. A method of detecting the presence or absence of multi-drug resistant *Mycobacterium sp.* in a sample, comprising:-
(a) contacting a set of probes according to any of Claims 1-13 with a nucleic acid-containing sample wherein, once a probe is bound to *Mycobacterium sp.* nucleic acid in the sample, a detectable signal is provided; and
(b) detecting said detectable signal;
wherein said probes are immobilised onto a solid support.

15. A method according to Claim 14, wherein the presence of multi-drug resistant *Mycobacterium sp.* in said sample is detected by:
(i) detecting a detectable signal provided by probes 2 and 4 and their respective bound target *Mycobacterium sp.* nucleic acid sequences in the sample; and
(ii) detecting the absence of a detectable signal provided by probes 1, 3, 5, 6, 7, 8, 9 and 10 and their respective target *Mycobacterium sp.* nucleic acid sequences.

16. A method according to Claim 14, wherein the absence of multi-drug resistant *Mycobacterium sp.* from said sample is detected by:
(i) detecting a detectable signal provided by probes 1, 3, 5, 6, 7, 8, 9 and 10 and their respective bound target *Mycobacterium sp.* nucleic acid sequence in the sample: and
(ii) detecting the absence of a detectable signal provided by probes 2 and 4 and their respective target *Mycobacterium sp.* nucleic acid sequences.

17. A method according to any of Claims 14-16, further comprising the step of amplifying *Mycobacterium sp.* nucleic acid in the sample prior to detection of signal.

18. A method according to Claim 17, wherein the amplification step is carried out prior to contacting the set of probes with the nucleic acid-containing sample.

19. A method according to Claim 17 or 18, wherein said amplification step comprises the step of labeling the *Mycobacterium sp.* nucleic acid.

20. A method according to any of Claims 14-19, wherein said detectable signal is a fluorescence signal.

21. A method according to any of Claims 14-20, wherein said sample is a clinical sample.

22. A method according to any of Claims 14-21 wherein said multi-drug resistant *Mycobacterium sp.* is a member of the *Mycobacterium tuberculosis* complex (MTC).

23. A method according to Claim 22 wherein said multi-drug resistant *Mycobacterium sp.* is *Mycobacterium tuberculosis.*

24. A kit for detection of multi-drug resistant *Mycobacterium sp.* nucleic acid comprising a set of probes according to any of Claims 1-13.

## Patentansprüche

1. Set von Nukleinsäure-Sonden zur Verwendung in einem Assay zum Nachweisen von gegen mehrere Arzneistoffe resistente Mycobacterium sp. in einer Probe, wobei das Set einschließt:
Sonde 1, umfassend eine Nukleinsäure-Sequenz von 10 Nukleotiden, die an eine erste Target-Sequenz ACCAGCGGCA, oder an das Komplement davon bindet;
Sonde 2, umfassend eine Nukleinsäure-Sequenz von 10 Nukleotiden, die an eine zweite Target-Sequenz GCCGGTGGTG, oder an das Komplement davon bindet;
Sonde 3, umfassend eine Nukleinsäure-Sequenz von 10 Nukleotiden, die an eine dritte Target-Sequenz TATCGTCTCG, oder an das Komplement davon bindet;
Sonde 4, umfassend eine Nukleinsäure-Sequenz von 10 Nukleotiden, die an eine vierte Target-Sequenz TATCATCTCG, oder an das Komplement davon bindet;
Sonde 5, umfassend eine Nukleinsäure-Sequenz von 10 Nukleotiden, die an eine fünfte Target-Sequenz GAATTGGCTC, oder an das Komplement davon bindet;
Sonde 6, umfassend eine Nukleinsäure-Sequenz von 10 Nukleotiden, die an eine sechste Target-Sequenz CTGGTCCATG, oder an das Komplement davon bindet;
Sonde 7, umfassend eine Nukleinsäure-Sequenz von 10 Nukleotiden, die an eine siebte Target-Sequenz GGTTGTTCTG, oder an das Komplement davon bindet;
Sonde 8, umfassend eine Nukleinsäure-Sequenz von 10 Nukleotiden, die an eine achte Target-Sequenz CCCGACAGCG, oder an das Komplement davon bindet;
Sonde 9, umfassend eine Nukleinsäure-Sequenz von 10 Nukleotiden, die an eine neunte Target-Sequenz GCTTGTGGGT, oder an das Komplement davon bindet; und Sonde 10, umfassend eine Nukleinsäure-Sequenz von 10 Nukleotiden, die an eine zehnte Target-Sequenz CCAGTGCCGA, oder an das Komplement davon bindet;
wobei jede von den Sonden 18-25 Nukleotide lang ist; und wobei bei Verwendung, wenn eine Sonde einmal an eine jeweilige Target-Sequenz gebunden ist, ein nachweisbares Signal bereitgestellt wird.

2. Set von Sonden nach Anspruch 1, wobei das Set einschließt:-
Sonde 1, umfassend eine Nukleinsäure-Sequenz von 15 Nukleotiden, die an eine erste Target-Sequenz ATCACCAGCGGCATC, oder an das Komplement davon bindet;
Sonde 2, umfassend eine Nukleinsäure-Sequenz von 15 Nukleotiden, die an eine zweite Target-Sequenz GATGCCGGTGGTGTA, oder an das Komplement davon bindet;
Sonde 3, umfassend eine Nukleinsäure-Sequenz von 15 Nukleotiden, die an eine dritte Target-Sequenz ACCTATCGTCTCGCC, oder an das Komplement davon bindet;
Sonde 4, umfassend eine Nukleinsäure-Sequenz von 15 Nukleotiden, die an eine vierte Target-Sequenz ACCTATCATCTCGCC, oder an das Komplement davon bindet;
Sonde 5, umfassend eine Nukleinsäure-Sequenz von 15 Nukleotiden, die an eine fünfte Target-Sequenz ATGAATTGGCTCAGC, oder an das Komplement davon bindet;
Sonde 6, umfassend eine Nukleinsäure-Sequenz von 15 Nukleotiden, die an eine sechste Target-Sequenz GTTCTGGTCCATGAA, oder an das Komplement davon bindet;
Sonde 7, umfassend eine Nukleinsäure-Sequenz von 15 Nukleotiden, die an eine siebte Target-Sequenz GCGGGTTGTTCTGGT, oder an das Komplement davon bindet;
Sonde 8, umfassend eine Nukleinsäure-Sequenz von 15 Nukleotiden, die an eine achte Target-Sequenz AACCCCGACAGCGGG, oder an das Komplement davon bindet;
Sonde 9, umfassend eine Nukleinsäure-Sequenz von 15 Nukleotiden, die an eine neunte Target-Sequenz GGCGCTTGTGGGTCA, oder an das Komplement davon bindet;
Sonde 10, umfassend eine Nukleinsäure-Sequenz von 15 Nukleotiden, die an eine zehnte Target-Sequenz GCCCCAGTGCCGACA, oder an das Komplement davon bindet.

3. Set von Sonden nach Anspruch 1 oder Anspruch 2, wobei das Set einschließt:-
Sonde 1, umfassend die Sequenz TGCCGCTGGT, oder eine Sequenz mit mindestens 90 % Sequenz-Identität dazu;
Sonde 2, umfassend die Sequenz CACCACCGGC, oder eine Sequenz mit mindestens 90 % Sequenz-Identität dazu;
Sonde 3, umfassend die Sequenz CGAGACGATA, oder eine Sequenz mit mindestens 90 % Sequenz-Identität dazu;
Sonde 4, umfassend die Sequenz CGAGATGATA, oder eine Sequenz mit mindestens 90 % Sequenz-Identität dazu;
Sonde 5, umfassend die Sequenz GAGCCAATTC, oder eine Sequenz mit mindestens 90 % Sequenz-Identität dazu;
Sonde 6, umfassend die Sequenz CATGGACCAG, oder eine Sequenz mit mindestens 90 % Sequenz-Identität dazu;
Sonde 7, umfassend die Sequenz CAGAACAACC, oder eine Sequenz mit mindestens 90 % Sequenz-Identität dazu;
Sonde 8, umfassend die Sequenz CGCTGTCGGG, oder eine Sequenz mit mindestens 90 % Sequenz-Identität dazu;
Sonde 9, umfassend die Sequenz ACCCACAAGC, oder eine Sequenz mit mindestens 90 % Sequenz-Identität dazu;
Sonde 10, umfassend die Sequenz TCGGCACTGG, oder eine Sequenz mit mindestens 90 % Sequenz-Identität dazu;
wobei die unterstrichenen Nukleotide innerhalb der Sequenzen von Sonden 1, 2, 3 und 4 essenziell sind, und nicht durch ein beliebiges anderes Nukleotid substituiert bzw. ersetzt sein können.

4. Set von Sonden nach Anspruch 3, wobei:-
Sonde 1 die Sequenz GATGCCGCTGGTGAT, oder eine Sequenz mit mindestens 90 % Sequenz-Identität dazu umfasst;
Sonde 2 die Sequenz ATCACCACCGGCATC, oder eine Sequenz mit mindestens 90 % Sequenz-Identität dazu umfasst;
Sonde 3 die Sequenz GGCGAGACGATAGGT, oder eine Sequenz mit mindestens 90 % Sequenz-Identität dazu umfasst;
Sonde 4 die Sequenz GGCGAGATGATAGGT, oder eine Sequenz mit mindestens 90 % Sequenz-Identität dazu umfasst;
Sonde 5 die Sequenz AGCTGAGCCAATTCATG, oder eine Sequenz mit mindestens 90 % Sequenz-Identität dazu umfasst;
Sonde 6 die Sequenz AATTCATGGACCAGAACA, oder eine Sequenz mit mindestens 90 % Sequenz-Identität dazu umfasst;
Sonde 7 die Sequenz ACCAGAACAACCCGC, oder eine Sequenz mit mindestens 90 % Sequenz-Identität dazu umfasst;
Sonde 8 die Sequenz ACCCGCTGTCGGGGTT, oder eine Sequenz mit mindestens 90 % Sequenz-Identität dazu umfasst;
Sonde 9 die Sequenz TGACCCACAAGCGCC, oder eine Sequenz mit mindestens 90 % Sequenz-Identität dazu umfasst;
Sonde 10 die Sequenz CTGTCGGCACTGGGGCC, oder eine Sequenz mit mindestens 90 % Sequenz-Identität dazu umfasst;
wobei die unterstrichenen Nukleotide innerhalb der Sequenzen von Sonden 1, 2, 3 und 4 essenziell sind, und nicht durch ein beliebiges anderes Nukleotid substituiert bzw. ersetzt sein können.

5. Set von Sonden nach einem vorangehenden Anspruch, wobei das Set einschließt:-
Sonde 1, umfassend die Sequenz SEQ ID Nr. 1, oder eine Sequenz mit mindestens 90 % Sequenz-Identität dazu;
Sonde 2, umfassend die Sequenz SEQ ID Nr. 2, oder eine Sequenz mit mindestens 90 % Sequenz-Identität dazu;
Sonde 3, umfassend die Sequenz SEQ ID Nr. 3, oder eine Sequenz mit mindestens 90 % Sequenz-Identität dazu;
Sonde 4, umfassend die Sequenz SEQ ID Nr. 4, oder eine Sequenz mit mindestens 90 % Sequenz-Identität dazu;
Sonde 5, umfassend die Sequenz SEQ ID Nr. 5, oder eine Sequenz mit mindestens 90 % Sequenz-Identität dazu;
Sonde 6, umfassend die Sequenz SEQ ID Nr. 6, oder eine Sequenz mit mindestens 90 % Sequenz-Identität dazu;
Sonde 7, umfassend die Sequenz SEQ ID Nr. 7, oder eine Sequenz mit mindestens 90 % Sequenz-Identität dazu;
Sonde 8, umfassend die Sequenz SEQ ID Nr. 8, oder eine Sequenz mit mindestens 90 % Sequenz-Identität dazu;
Sonde 9, umfassend die Sequenz SEQ ID Nr. 9, oder eine Sequenz mit mindestens 90 % Sequenz-Identität dazu;
Sonde 10, umfassend die Sequenz SEQ ID Nr. 10, oder eine Sequenz mit mindestens 90 % Sequenz-Identität dazu;
wobei Nukleotid-Rest 11 innerhalb von SEQ ID Nrn. 1 und 2 essenziell ist und nicht durch ein beliebiges anderes Nukleotid substituiert bzw. ersetzt sein kann;
und wobei Nukleotid-Rest 8 innerhalb von SEQ ID Nrn. 3 und 4 essenziell ist und nicht durch ein beliebiges anderes Nukleotid substituiert bzw. ersetzt sein kann.

6. Set von Nukleinsäure-Sonden zur Verwendung in einem Assay zum Nachweisen von gegen mehrere Arzneistoffe resistente Mycobacterium sp. in einer Probe, wobei das Set einschließt:-
Sonde 1, umfassend eine Nukleinsäure-Sequenz von 10 Nukleotiden, die an eine erste Target-Sequenz ACCAGCGGCA, oder an das Komplement davon bindet;
Sonde 2, umfassend eine Nukleinsäure-Sequenz von 10 Nukleotiden, die an eine zweite Target-Sequenz GCCGGTGGTG, oder an das Komplement davon bindet;
Sonde 5, umfassend eine Nukleinsäure-Sequenz von 10 Nukleotiden, die an eine fünfte Target-Sequenz GAATTGGCTC, oder an das Komplement davon bindet;
Sonde 6, umfassend eine Nukleinsäure-Sequenz von 10 Nukleotiden, die an eine sechste Target-Sequenz CTGGTCCATG, oder an das Komplement davon bindet;
Sonde 7, umfassend eine Nukleinsäure-Sequenz von 10 Nukleotiden, die an eine siebte Target-Sequenz GGTTGTTCTG, oder an das Komplement davon bindet;
Sonde 8, umfassend eine Nukleinsäure-Sequenz von 10 Nukleotiden, die an eine achte Target-Sequenz CCCGACAGCG, oder an das Komplement davon bindet;
Sonde 9, umfassend eine Nukleinsäure-Sequenz von 10 Nukleotiden, die an eine neunte Target-Sequenz GCTTGTGGGT, oder an das Komplement davon bindet; und Sonde 10, umfassend eine Nukleinsäure-Sequenz von 10 Nukleotiden, die an eine zehnte Target-Sequenz CCAGTGCCGA, oder an das Komplement davon bindet;
wobei jede von den Sonden 18-25 Nukleotide lang ist;
und wobei bei Verwendung, wenn eine Sonde einmal an eine jeweilige Target-Sequenz gebunden ist, ein nachweisbares Signal bereitgestellt wird.

7. Set von Sonden nach Anspruch 6, wobei das Set einschließt:-
Sonde 1, umfassend die Sequenz TGCCGCTGGT, oder eine Sequenz mit mindestens 90 % Sequenz-Identität dazu;
Sonde 2, umfassend die Sequenz CACCACCGGC, oder eine Sequenz mit mindestens 90 % Sequenz-Identität dazu;
Sonde 5, umfassend die Sequenz GAGCCAATTC, oder eine Sequenz mit mindestens 90 % Sequenz-Identität dazu;
Sonde 6, umfassend die Sequenz CATGGACCAG, oder eine Sequenz mit mindestens 90 % Sequenz-Identität dazu;
Sonde 7, umfassend die Sequenz CAGAACAACC, oder eine Sequenz mit mindestens 90 % Sequenz-Identität dazu;
Sonde 8, umfassend die Sequenz CGCTGTCGGG, oder eine Sequenz mit mindestens 90 % Sequenz-Identität dazu;
Sonde 9, umfassend die Sequenz ACCCACAAGC, oder eine Sequenz mit mindestens 90 % Sequenz-Identität dazu;
Sonde 10, umfassend die Sequenz TCGGCACTGG, oder eine Sequenz mit mindestens 90 % Sequenz-Identität dazu;
wobei die unterstrichenen Nukleotide innerhalb der Sequenzen von Sonden 1 und 2 essenziell sind, und nicht durch ein beliebiges anderes Nukleotid substituiert bzw. ersetzt sein kann.

8. Set von Sonden nach einem vorangehenden Anspruch, wobei eine oder mehrere von Sonden 5, 6, 7, 8, 9 und 10 durch eine oder mehrere Sonden mit mindestens 90 % Sequenz-Identität zu einer Sequenz, ausgewählt aus SEQ ID Nrn.: 11-32, substituiert bzw. ersetzt ist.

9. Set von Sonden nach einem von Ansprüchen 1-7, weiterhin umfassend eine oder mehrere Sonden mit mindestens 90 % Sequenz-Identität zu einer Sequenz, ausgewählt aus SEQ ID Nrn.: 11-32.

10. Set von Sonden nach einem vorangehenden Anspruch, wobei die Sonden auf einem festen Träger immobilisiert sind.

11. Set von Sonden nach einem vorangehenden Anspruch, wobei die Sonden jede einen 3' Poly-T-Tail aufweisen.

12. Set von Sonden nach einem vorangehenden Anspruch, wobei die gegen mehrere Arzneistoffe resistente Mycobacterium sp. gegen mehrere Arzneistoffe resistente Mitglieder von dem Mycobacterium tuberculosis-Komplex (MTC) sind.

13. Set von Sonden nach Anspruch 10, wobei die gegen mehrere Arzneistoffe resistente Mycobacterium sp. gegen mehrere Arzneistoffe resistentes Mycobacterium tuberculosis sind.

14. Verfahren zum Nachweisen für das Vorliegen oder die Abwesenheit von gegen mehrere Arzneistoffe resistente Mycobacterium sp. in einer Probe, umfassend:-
(a) In-Kontakt-Bringen eines Sets von Sonden nach einem von Ansprüchen 1-13 mit einer Nukleinsäure-enthaltenden Probe, worin, wenn eine Sonde einmal an Mycobacterium sp.-Nukleinsäure in der Probe gebunden ist, ein nachweisbares Signal bereitgestellt wird; und
(b) Nachweisen des nachweisbaren Signals;
wobei die Sonden auf einem festen Träger immobilisiert sind.

15. Verfahren nach Anspruch 14, wobei das Vorliegen von gegen mehrere Arzneistoffe resistente Mycobacterium sp. in der Probe nachgewiesen wird durch:
(i) Nachweisen eines nachweisbaren Signals, das durch Sonden 2 und 4 und deren jeweilige gebundene Target-Mycobacterium sp.-Nukleinsäure-Sequenzen in der Probe bereitgestellt wird; und
(ii) Nachweisen der Abwesenheit von einem nachweisbaren Signal, das durch Sonden 1, 3, 5, 6, 7, 8, 9 und 10 und deren jeweilige Target-Mycobacterium sp.-Nukleinsäure-Sequenzen bereitgestellt wird.

16. Verfahren nach Anspruch 14, wobei die Abwesenheit von gegen mehrere Arzneistoffe resistente Mycobacterium sp. von der Probe nachgewiesen wird durch:
(i) Nachweisen eines nachweisbaren Signals, das durch Sonden 1, 3, 5, 6, 7, 8, 9 und 10 und deren jeweilige gebundene Target-Mycobacterium sp.-Nuklein-säure-Sequenz in der Probe bereitgestellt wird; und
(ii) Nachweisen der Abwesenheit von einem nachweisbaren Signal, das durch Sonden 2 und 4 und deren jeweilige Target-Mycobacterium sp.-Nukleinsäure-Sequenzen bereitgestellt wird.

17. Verfahren nach einem der Ansprüche 14-16, weiterhin umfassend den Schritt des Verstärkens bzw. Amplifizierens von Mycobacterium sp.-Nukleinsäure in der Probe vor dem Nachweis des Signals.

18. Verfahren nach Anspruch 17, wobei der Verstärkungs- bzw. Amplifizierungs-Schritt vor dem In-Kontakt-Bringen des Sets von Sonden mit der Nukleinsäure-enthaltenden Probe ausgeführt wird.

19. Verfahren nach Anspruch 17 oder 18, wobei der Verstärkungs- bzw. Amplifizierungs-Schritt den Schritt des Markierens von der Mycobacterium sp.-Nukleinsäure umfasst.

20. Verfahren nach einem der Ansprüche 14-19, wobei das nachweisbare Signal ein Fluoreszenz-Signal ist.

21. Verfahren nach einem der Ansprüche 14-20, wobei die Probe eine klinische Probe ist.

22. Verfahren nach einem der Ansprüche 14-21, wobei die gegen mehrere Arzneistoffe resistente Mycobacterium sp. ein Mitglied von dem Mycobacterium tuberculosis-Komplex (MTC) ist.

23. Verfahren nach Anspruch 22, wobei die gegen mehrere Arzneistoffe resistente Mycobacterium sp. Mycobacterium tuberculosis ist.

24. Kit zum Nachweis von gegen mehrere Arzneistoffe resistenter Mycobacterium sp.-Nukleinsäure, umfassend ein Set von Sonden nach einem von Ansprüchen 1-13.

## Revendications

1. Jeu de sondes d'acide nucléique destiné à être utilisé dans un test de détection de *Mycobacterium sp.* présentant une résistance multi-médicamenteuse dans un échantillon, lequel jeu comprend :
la sonde 1 comprenant une séquence d'acide nucléique de 10 nucléotides qui se lie à une première séquence cible ACCAGCGGCA, ou à son complément ;
la sonde 2 comprenant une séquence d'acide nucléique de 10 nucléotides qui se lie à une deuxième séquence cible GCCGGTGGTG, ou à son complément ;
la sonde 3 comprenant une séquence d'acide nucléique de 10 nucléotides qui se lie à une troisième séquence cible TATCGTCTCG, ou à son complément ;
la sonde 4 comprenant une séquence d'acide nucléique de 10 nucléotides qui se lie à une quatrième séquence cible TATCATCTCG, ou à son complément ;
la sonde 5 comprenant une séquence d'acide nucléique de 10 nucléotides qui se lie à une cinquième séquence cible GAATTGGCTC, ou à son complément ;
la sonde 6 comprenant une séquence d'acide nucléique de 10 nucléotides qui se lie à une sixième séquence cible CTGGTCCATG, ou à son complément ;
la sonde 7 comprenant une séquence d'acide nucléique de 10 nucléotides qui se lie à une septième séquence cible GGTTGTTCTG, ou à son complément ;
la sonde 8 comprenant une séquence d'acide nucléique de 10 nucléotides qui se lie à une huitième séquence cible CCCGACAGCG, ou à son complément ;
la sonde 9 comprenant une séquence d'acide nucléique de 10 nucléotides qui se lie à une neuvième séquence cible GCTTGTGGGT, ou à son complément ; et la sonde 10 comprenant une séquence d'acide nucléique de 10 nucléotides qui se lie à une dixième séquence cible CCAGTGCCGA, ou à son complément ;
chacune desdites sondes étant d'une longueur de 18 à 25 nucléotides ;
où un signal détectable est délivré durant l'utilisation dès qu'une sonde est liée à une séquence cible respective.

2. Jeu de sondes selon la revendication 1, lequel jeu comprend :
la sonde 1 comprenant une séquence d'acide nucléique de 15 nucléotides qui se lie à une première séquence cible ATCACCAGCGGCATC, ou à son complément ;
la sonde 2 comprenant une séquence d'acide nucléique de 15 nucléotides qui se lie à une deuxième séquence cible GATGCCGGTGGTGTA, ou à son complément ;
la sonde 3 comprenant une séquence d'acide nucléique de 15 nucléotides qui se lie à une troisième séquence cible ACCTATCGTCTCGCC, ou à son complément ;
la sonde 4 comprenant une séquence d'acide nucléique de 15 nucléotides qui se lie à une quatrième séquence cible ACCTATCATCTCGCC, ou à son complément ;
la sonde 5 comprenant une séquence d'acide nucléique de 15 nucléotides qui se lie à une cinquième séquence cible ATGAATTGGCTCAGC, ou à son complément ;
la sonde 6 comprenant une séquence d'acide nucléique de 15 nucléotides qui se lie à une sixième séquence cible GTTCTGGTCCATGAA, ou à son complément ;
la sonde 7 comprenant une séquence d'acide nucléique de 15 nucléotides qui se lie à une septième séquence cible GCGGGTTGTTCTGGT, ou à son complément ;
la sonde 8 comprenant une séquence d'acide nucléique de 15 nucléotides qui se lie à une huitième séquence cible AACCCCGACAGCGGG, ou à son complément ;
la sonde 9 comprenant une séquence d'acide nucléique de 15 nucléotides qui se lie à une neuvième séquence cible GGCGCTTGTGGGTCA, ou à son complément ;
la sonde 10 comprenant une séquence d'acide nucléique de 15 nucléotides qui se lie à une dixième séquence cible GCCCCAGTGCCGACA, ou à son complément.

3. Jeu de sondes selon la revendication 1 ou la revendication 2, lequel jeu comprend : la sonde 1 comprenant la séquence TGCCGCTGGT, ou une séquence présentant une identité de séquence d'au moins 90 % avec celle-ci ;
la sonde 2 comprenant la séquence CACCACCGGC, ou une séquence présentant une identité de séquence d'au moins 90 % avec celle-ci ;
la sonde 3 comprenant la séquence CGAGACGATA, ou une séquence présentant une identité de séquence d'au moins 90 % avec celle-ci ;
la sonde 4 comprenant la séquence CGAGATGATA, ou une séquence présentant une identité de séquence d'au moins 90 % avec celle-ci ;
la sonde 5 comprenant la séquence GAGCCAATTC, ou une séquence présentant une identité de séquence d'au moins 90 % avec celle-ci ;
la sonde 6 comprenant la séquence CATGGACCAG, ou une séquence présentant une identité de séquence d'au moins 90 % avec celle-ci ;
la sonde 7 comprenant la séquence CAGAACAACC, ou une séquence présentant une identité de séquence d'au moins 90 % avec celle-ci ;
la sonde 8 comprenant la séquence CGCTGTCGGG, ou une séquence présentant une identité de séquence d'au moins 90 % avec celle-ci ;
la sonde 9 comprenant la séquence ACCCACAAGC, ou une séquence présentant une identité de séquence d'au moins 90 % avec celle-ci ;
la sonde 10 comprenant la séquence TCGGCACTGG, ou une séquence présentant une identité de séquence d'au moins 90 % avec celle-ci ;
les nucléotides soulignés dans les séquences des sondes 1, 2, 3 et 4 étant essentiels, et ne pouvant être remplacés par aucun autre nucléotide.

4. Jeu de sondes selon la revendication 3, dans lequel :
la sonde 1 comprend la séquence GATGCCGCTGGTGAT, ou une séquence présentant une identité de séquence d'au moins 90 % avec celle-ci ;
la sonde 2 comprend la séquence ATCACCACCGGCATC, ou une séquence présentant une identité de séquence d'au moins 90 % avec celle-ci ;
la sonde 3 comprend la séquence GGCGAGACGATAGGT, ou une séquence présentant une identité de séquence d'au moins 90 % avec celle-ci ;
la sonde 4 comprend la séquence GGCGAGATGATAGGT, ou une séquence présentant une identité de séquence d'au moins 90 % avec celle-ci ;
la sonde 5 comprend la séquence AGCTGAGCCAATTCATG, ou une séquence présentant une identité de séquence d'au moins 90 % avec celle-ci ;
la sonde 6 comprend la séquence AATTCATGGACCAGAACA, ou une séquence présentant une identité de séquence d'au moins 90 % avec celle-ci ;
la sonde 7 comprend la séquence ACCAGAACAACCCGC, ou une séquence présentant une identité de séquence d'au moins 90 % avec celle-ci ;
la sonde 8 comprend la séquence ACCCGCTGTCGGGGTT, ou une séquence présentant une identité de séquence d'au moins 90 % avec celle-ci ;
la sonde 9 comprend la séquence TGACCCACAAGCGCC, ou une séquence présentant une identité de séquence d'au moins 90 % avec celle-ci ;
la sonde 10 comprend la séquence CTGTCGGCACTGGGGCC, ou une séquence présentant une identité de séquence d'au moins 90 % avec celle-ci ;
les nucléotides soulignés dans les séquences des sondes 1, 2, 3 et 4 étant essentiels, et ne pouvant être remplacés par aucun autre nucléotide.

5. Jeu de sondes selon l'une quelconque des revendications précédentes, lequel jeu comprend :
la sonde 1 comprenant la séquence SEQ ID NO : 1, ou une séquence présentant une identité de séquence d'au moins 90 % avec celle-ci ;
la sonde 2 comprenant la séquence SEQ ID NO : 2, ou une séquence présentant une identité de séquence d'au moins 90 % avec celle-ci ;
la sonde 3 comprenant la séquence SEQ ID NO : 3, ou une séquence présentant une identité de séquence d'au moins 90 % avec celle-ci ;
la sonde 4 comprenant la séquence SEQ ID NO : 4, ou une séquence présentant une identité de séquence d'au moins 90 % avec celle-ci ;
la sonde 5 comprenant la séquence SEQ ID NO : 5, ou une séquence présentant une identité de séquence d'au moins 90 % avec celle-ci ;
la sonde 6 comprenant la séquence SEQ ID NO : 6, ou une séquence présentant une identité de séquence d'au moins 90 % avec celle-ci ;
la sonde 7 comprenant la séquence SEQ ID NO : 7, ou une séquence présentant une identité de séquence d'au moins 90 % avec celle-ci ;
la sonde 8 comprenant la séquence SEQ ID NO : 8, ou une séquence présentant une identité de séquence d'au moins 90 % avec celle-ci ;
la sonde 9 comprenant la séquence SEQ ID NO : 9, ou une séquence présentant une identité de séquence d'au moins 90 % avec celle-ci ;
la sonde 10 comprenant la séquence SEQ ID NO : 10, ou une séquence présentant une identité de séquence d'au moins 90 % avec celle-ci ;
le résidu nucléotidique 11 dans les SEQ ID NO : 1 et 2 étant essentiel et ne pouvant être remplacé par aucun autre nucléotide ;
et le résidu nucléotidique 8 dans les SEQ ID NO : 3 et 4 étant essentiel et ne pouvant être remplacé par aucun autre nucléotide.

6. Jeu de sondes d'acide nucléique destiné à être utilisé dans un test de détection de *Mycobacterium sp.* présentant une résistance multi-médicamenteuse dans un échantillon, lequel jeu comprend :
la sonde 1 comprenant une séquence d'acide nucléique de 10 nucléotides qui se lie à une première séquence cible ACCAGCGGCA, ou à son complément ;
la sonde 2 comprenant une séquence d'acide nucléique de 10 nucléotides qui se lie à une deuxième séquence cible GCCGGTGGTG, ou à son complément ;
la sonde 5 comprenant une séquence d'acide nucléique de 10 nucléotides qui se lie à une cinquième séquence cible GAATTGGCTC, ou à son complément ;
la sonde 6 comprenant une séquence d'acide nucléique de 10 nucléotides qui se lie à une sixième séquence cible CTGGTCCATG, ou à son complément ;
la sonde 7 comprenant une séquence d'acide nucléique de 10 nucléotides qui se lie à une septième séquence cible GGTTGTTCTG, ou à son complément ;
la sonde 8 comprenant une séquence d'acide nucléique de 10 nucléotides qui se lie à une huitième séquence cible CCCGACAGCG, ou à son complément ;
la sonde 9 comprenant une séquence d'acide nucléique de 10 nucléotides qui se lie à une neuvième séquence cible GCTTGTGGGT, ou à son complément ; et la sonde 10 comprenant une séquence d'acide nucléique de 10 nucléotides qui se lie à une dixième séquence cible CCAGTGCCGA, ou à son complément ;
où chacune desdites sondes est d'une longueur de 18 à 25 nucléotides ;
et où un signal détectable est délivré durant l'utilisation une fois qu'une sonde est liée à une séquence cible respective.

7. Jeu de sondes selon la revendication 6, lequel jeu comprend :
la sonde 1 comprenant la séquence TGCCGCTGGT, ou une séquence présentant une identité de séquence d'au moins 90 % avec celle-ci ;
la sonde 2 comprenant la séquence CACCACCGGC, ou une séquence présentant une identité de séquence d'au moins 90 % avec celle-ci ;
la sonde 5 comprenant la séquence GAGCCAATTC, ou une séquence présentant une identité de séquence d'au moins 90 % avec celle-ci ;
la sonde 6 comprenant la séquence CATGGACCAG, ou une séquence présentant une identité de séquence d'au moins 90 % avec celle-ci ;
la sonde 7 comprenant la séquence CAGAACAACC, ou une séquence présentant une identité de séquence d'au moins 90 % avec celle-ci ;
la sonde 8 comprenant la séquence CGCTGTCGGG, ou une séquence présentant une identité de séquence d'au moins 90 % avec celle-ci ;
la sonde 9 comprenant la séquence ACCCACAAGC, ou une séquence présentant une identité de séquence d'au moins 90 % avec celle-ci ;
la sonde 10 comprenant la séquence TCGGCACTGG, ou une séquence présentant une identité de séquence d'au moins 90 % avec celle-ci ;
les nucléotides soulignés dans les séquences des sondes 1 et 2 étant essentiels, et ne pouvant être remplacés par aucun autre nucléotide.

8. Jeu de sondes selon l'une quelconque des revendications précédentes, dans lequel une ou plusieurs des sondes 5, 6, 7, 8, 9 et 10 est remplacée par une ou plusieurs des sondes ayant une identité de séquence d'au moins 90 % avec une séquence choisie parmi SEQ ID NO : 11 à 32.

9. Jeu de sondes selon l'une quelconque des revendications 1 à 7, comprenant en outre une ou plusieurs sondes ayant une identité de séquence d'au moins 90 % avec une séquence choisie parmi SEQ ID NO : 11 à 32.

10. Jeu de sondes selon l'une quelconque des revendications précédentes, dans lequel lesdites sondes sont immobilisées sur un support solide.

11. Jeu de sondes selon l'une quelconque des revendications précédentes, dans lequel lesdites sondes ont chacune une queue poly-T en 3'.

12. Jeu de sondes selon l'une quelconque des revendications précédentes, dans lequel lesdites mycobactéries *Mycobacterium sp.* présentant une résistance multi-médicamenteuse sont des membres présentant une résistance multi-médicamenteuse du complexe *Mycobacterium Tuberculosis* (MTC).

13. Jeu de sondes selon la revendication 10, dans lequel lesdites mycobactéries *Mycobacterium sp.* présentant une résistance multi-médicamenteuse sont des *Mycobacterium Tuberculosis* présentant une résistance multi-médicamenteuse.

14. Procédé de détection de la présence ou de l'absence de *Mycobacterium sp.* présentant une résistance multi-médicamenteuse dans un échantillon, qui comprend :
(a) la mise en contact d'un jeu de sondes selon l'une quelconque des revendications 1 à 13, avec un échantillon contenant un acide nucléique, un signal détectable étant délivré dès qu'une sonde se lie à l'acide nucléique de *Mycobacterium sp.* dans l'échantillon ; et
(b) la détection dudit signal détectable ;
lesdites sondes étant immobilisées sur un support solide.

15. Procédé selon la revendication 14, dans lequel la présence de *Mycobacterium sp.* présentant une résistance multi-médicamenteuse dans ledit échantillon est détectée par :
(i) la détection d'un signal détectable fourni par les sondes 2 et 4 et leurs séquences cibles respectives d'acide nucléique de *Mycobacterium sp.* liées dans l'échantillon ; et
(ii) la détection de l'absence d'un signal détectable fourni par les sondes 1, 3, 5, 6, 7, 8, 9 et 10 et leurs séquences cibles respectives d'acide nucléique de *Mycobacterium sp..*

16. Procédé selon la revendication 14, dans lequel l'absence de *Mycobacterium sp.* présentant une résistance multi-médicamenteuse dans ledit échantillon est détectée par :
(i) la détection d'un signal détectable fourni par les sondes 1, 3, 5, 6, 7, 8, 9 et 10 et leurs séquences cibles respectives d'acide nucléique de *Mycobacterium sp.* liées dans l'échantillon ; et
(ii) la détection de l'absence d'un signal détectable fourni par les sondes 2 et 4 et leurs séquences cibles respectives d'acide nucléique de *Mycobacterium sp..*

17. Procédé selon l'une quelconque des revendications 14 à 16, comprenant en outre l'étape consistant à amplifier l'acide nucléique de *Mycobacterium sp.* dans l'échantillon avant la détection du signal.

18. Procédé selon la revendication 17, dans lequel l'étape d'amplification est réalisée avant la mise en contact du jeu de sondes avec l'échantillon contenant l'acide nucléique.

19. Procédé selon la revendication 17 ou la revendication 18, dans lequel ladite étape d'amplification comprend l'étape consistant à marquer l'acide nucléique de *Mycobacterium sp.*

20. Procédé selon l'une quelconque des revendications 14 à 19, dans lequel ledit signal détectable est un signal fluorescent.

21. Procédé selon l'une quelconque des revendications 14 à 20, dans lequel ledit échantillon est un échantillon clinique.

22. Procédé selon l'une quelconque des revendications 14 à 21, dans lequel ladite mycobactérie *Mycobacterium sp.* présentant une résistance multi-médicamenteuse est un membre du complexe *Mycobacterium Tuberculosis* (MTC).

23. Procédé selon la revendication 22, dans lequel ladite mycobactérie *Mycobacterium sp.* présentant une résistance multi-médicamenteuse est *Mycobacterium Tuberculosis.*

24. Kit de détection de l'acide nucléique de *Mycobacterium sp.* présentant une résistance multi-médicamenteuse qui comprend un jeu de sondes selon l'une quelconque des revendications 1 à 13.
